# EUROPÄISCHE PATENTSCHRIFT

(11) **EP 3 079 653 B1**
(45) Veröffentlichungstag und Bekanntmachung des Hinweises auf die Patenterteilung: **22.08.2018**
(21) Anmeldenummer: 14802031.6
(22) Anmeldetag: 20.11.2014
(51) Int. Cl.: A61K 8/46, A61K 8/19, A61K 8/23, A61K 8/43, A61Q 5/04, A61Q 5/06, A61Q 5/12

(54) **VERFAHREN ZUM GLÄTTEN, KONDITIONIEREN UND FÄRBEN VON HAAREN, INSBESONDERE VON HAAREN MIT STARKER KRAUSE**
METHOD FOR STRAIGHTENING, CONDITIONING AND COLOURING HAIR, ESPECIALLY HAIR WITH SUBSTANTIAL CURL
PROCÉDÉ DESTINÉ AU LISSAGE, AU CONDITIONNEMENT ET À LA COLORATION DE CHEVEUX, NOTAMMENT DE CHEVEUX TRÈS FRISÉS

(30) Priorität: 12.12.2013 DE 102013225788
(43) Veröffentlichungstag der Anmeldung: 19.10.2016
(73) Patentinhaber: Henkel AG & Co. KGaA, 40589 Düsseldorf (DE)
(72) Erfinder: GOUTSIS, Konstantin, 41363 Jüchen (DE); WESER, Gabriele, 41472 Neuss (DE)
(86) Internationale Anmeldenummer: PCT/EP2014/075139
(87) Internationale Veröffentlichungsnummer: WO 2015/086283

(56) Entgegenhaltungen:
- FR-A1- 2 931 667
- JP-A- 2003 231 619
- US-A1- 2005 076 459

## Beschreibung

Gegenstand der vorliegenden Anmeldung ist ein Verfahren für die Behandlung von Haaren, insbesondere von Haaren mit starker Krause, welches es erlaubt, das Haar zu glätten, zu konditionieren und zu färben. Ein weiterer Gegenstand der vorliegenden Erfindung ist eine Mehrkomponenten-Verpackungseinheit (Kit), welche getrennt voneinander konfektioniert ein Glättungsmittel, ein Konditioniermittel und ein Färbemittel enthält. Das Färbemittel enthält mindestens einen direktziehenden Säurefarbstoff, und das Konditioniermittel und/oder das Färbemittel enthalten mindestens ein Reduktionsmittel.

Die Veränderung von Form und Farbe von keratinischen Fasern, insbesondere von Haaren, stellt einen wichtigen Bereich der modernen Kosmetik dar. Hierdurch kann das Erscheinungsbild der Haare sowohl aktuellen Modeströmungen als auch den individuellen Wünschen der einzelnen Person angepasst werden. Die Färbung, insbesondere die Abdeckung von ergrauten Haaren, wird von Menschen aller Kulturkreise angestrebt. Menschen aus Kulturen mit naturgegebenen krausen Haaren sind darüber hinaus oft auch auf der Suche nach Möglichkeiten, die Haare zu glätten. Haarglättungsmittel werden zur Entkräuselung von gelocktem Haar angewandt. Hierbei müssen die Haarglättungsmittel eine Reihe von Mindestanforderungen erfüllen: Mit den Mitteln muss sich - insbesondere bei Haaren mit sehr starker Krause, wie man sie beispielsweise bei afrikanischem Haar findet - eine wirksame Glättung des stark gekräuselten Haares bewirken lassen, wobei die Glättung unabhängig vom Haarzustand erfolgen sollte. Weiterhin müssen die Mittel temperaturstabil sein, sollten eine optimale Konsistenz besitzen, keine Hautirritationen hervorrufen und leicht wieder auswaschbar sein. Zusätzlich sollten die Mittel keine übermäßige Schädigung des Haares hervorrufen. Die am häufigsten angewandte Methode, sehr stark gekräuseltes Haar zu glätten, ist die Behandlung der Haare mit Präparaten auf Basis von Natrium-, Kalium-, Lithium und/oder Guanidiniumhydroxid. Bei Anwendung dieser Haarglättungsmittel werden die im Haar befindlichen Cystin-Bindungen gespalten, und etwa ein Drittel des Cystin-Gehaltes wird in Lanthionin umgewandelt. Da die mit den vorgenannten Hydroxiden durchgeführte Haarglättung die Haare starker Alkalität aussetzt, lässt sich die Schädigung der Haare oftmals nicht vermeiden.

Zur Veränderung der Haarfarbe kennt der Fachmann je nach Anforderung an die Färbung diverse Färbesysteme. Für permanente, intensive Färbungen mit entsprechenden Echtheitseigenschaften werden sogenannte Oxidationsfärbemittel verwendet. Solche Färbemittel enthalten üblicherweise Oxidationsfarbstoffvorprodukte, sogenannte Entwicklerkomponenten und Kupplerkomponenten, die unter dem Einfluss von Oxidationsmitteln wie beispielsweise Wasserstoffperoxid untereinander die eigentlichen Farbstoffe ausbilden. Oxidationsfärbemittel zeichnen sich durch hervorragende, lang anhaltende Färbeergebnisse aus, sind jedoch auch mit einem gewissen Ausmaß an Haarschädigung verbunden. Bei Einsatz von Oxidationsfärbemitteln in kombinierten Glättungs-Färbeverfahren würde eine Akkumulation der Schädigung auftreten, aus diesem Grund ist der Einsatz von Oxidationsfärbemitteln in Glättungs-Färbeverfahren nicht angezeigt.

Durch den Einsatz von direktziehenden Farbstoffen kann die Haarfarbe temporär verändert werden. Hierbei diffundieren bereits fertig ausgebildete Farbstoffe aus dem Färbemittel in die Haarfaser hinein. Im Vergleich zur oxidativen Haarfärbung weisen die mit direktziehenden Farbstoffen erhaltenen Färbungen eine geringere Haltbarkeit und schnellere Auswaschbarkbeit auf. Von Vorteil ist jedoch die geringere Haarschädigung der Färbung mit direktziehenden Farbstoffen.

Verfahren zur kombinierten Glättung und Färbung von Haaren sind im Stand der Technik bereits beschrieben. So offenbart beispielsweise WO 2013/098335 A2 ein Verfahren zum Glätten und Färben von Keratinfasern, in welchem Hydroxid-Basen und Farbstoffe gleichzeitig auf den Fasern zur Anwendung kommen. Die aus dem Stand der Technik bekannten Verfahren und Mittel sind jedoch oftmals mit einer hohen bis sehr hohen Haarschädigung verbunden. Weitere kombinierte Glättungs- und Färbeverfahren sind aus JP 2003 231619 A und US 2005/076459 A1 bekannt.

Der vorliegenden Erfindung lag die Aufgabe zugrunde, ein schädigungsarmes Verfahren zum Glätten und Färben von keratinischen Fasern bereit zu stellen. Durch den Einsatz dieses Verfahrens sollten sich insbesondere auch sehr stark gekräuselte Haare - wie beispielsweise afrikanisches Haar - wirkungsvoll glätten und färben lassen. Die Haare sollten durch dieses Verfahren möglichst wenig geschädigt werden.

Es hat sich nun herausgestellt, dass sich keratinische Fasern mit sehr starker Krause effektiv glätten und intensiv färben lassen, wenn sie einem speziellen Verfahren unterzogen werden,
welches eine alkalische Glättung, die Konditionierung der Fasern und die Färbung mit Säurefarbstoffen umfasst. Überraschenderweise lässt sich die Haarschädigung hierbei besonders stark minimieren, wenn das Konditioniermittel und/oder das Färbemittel ein Reduktionsmittel enthält.

Ein erster Gegenstand der vorliegenden Erfindung ist ein Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und/oder das Färbemittel (F) mindestens ein Reduktionsmittel aus der Gruppe Thio-Milchsäure, Thio-Glycolsäure, Cystein, Natriumsulfid, Natriumsulfit, Natriumthiosulfat, Natriumdithionit und/oder deren kosmetisch verträglichen Salze enthält und
- das Färbemittel (F) mindestens einen direktziehenden Säurefarbstoff enthält.

Unter keratinischen Fasern, keratinhaltigen Fasern oder Keratinfasern sind Pelze, Wolle, Federn und insbesondere menschliche Haare zu verstehen. Obwohl die erfindungsgemäßen Mittel in erster Linie zum Aufhellen und Färben von Keratinfasern geeignet sind, steht prinzipiell einer Verwendung auch auf anderen Gebieten nichts entgegen.

Kennzeichnend für das erfindungsgemäße Verfahren ist die Anwendung von Glättungsmittel (G), Konditioniermittel (K) und Färbemittel (F). Bei diesen drei Mitteln handelt es sich um drei verschiedene kosmetische Mittel, die alle wesentlichen Inhaltsstoffe jeweils in einem kosmetischen Träger enthalten. Bei dem kosmetischen Träger kann es sich um einen geeigneten wässrigen, alkoholischen oder wässrig-alkoholischen Träger handeln. Beispielsweise können das Glättungsmittel (G), das Konditioniermittel (K) und das Färbemittel (F) jeweils in Form einer Creme, einer Emulsion, eines Gels oder auch in Form einer tensidhaltigen schäumenden Lösung, wie beispielsweise eines Shampoos, eines Schaumaerosols, einer Schaumformulierung oder in Form einer anderen Zubereitung, die für die Anwendung auf dem Haar geeignet ist, auf die keratinischen Fasern aufgetragen werden. Das erfindungsgemäße Verfahren umfasst die folgenden Schritte in der angegebenen Reihenfolge:
A) Behandeln der Fasern mit einem Glättungsmittel (G),
B) Behandeln der Fasern mit einem Konditioniermittel (K),
C) Behandeln der Fasern mit einem Färbemittel (F).
Diese Ausführungsart des Verfahrens erlaubt die Glättung und Färbung der keratinischen Fasern mit sehr geringer Schädigung.

Im ersten Schritt (A) des erfindungsgemäßen Verfahrens wird ein kosmetisches Glättungsmittel (G) auf die Fasern appliziert. Das Glättungsmittel (G) wird für einen Zeitraum von 30 Sekunden bis 45 Minuten auf die Keratinfasern einwirken gelassen und im Anschluss daran wieder ausgespült. Im Anschluss daran (d.h. im zweiten Schritt (B)) folgt in einer Ausführungsform die Behandlung der Keratinfasern mit dem Konditioniermittel (K). Das Konditioniermittel (K) wird für einen Zeitraum von 30 Sekunden bis 45 Minuten auf die Keratinfasern einwirken gelassen, bevor es wieder ausgespült wird. Auf das Ausspülen des Konditioniermittels folgt daraufhin (im dritten Schritt (C)) die Färbung mit einem Färbemittel (F), welches mindestens einen Säurefarbstoff enthält. Das Färbemittel (F) wird für einen Zeitraum von 30 Sekunden bis 45 Minuten einwirken gelassen, bevor es wieder ausgespült wird.

Die Glättung der Keratinfasern kann während der Einwirkung des Glättungsmittels (G) auf die Keratinfasern oder kurz nach dem Auswaschen des Glättungsmittels (G) vorgenommen werden. Hierbei erfolgt die Glättung bevorzugt mechanisch, beispielsweise durch Glattziehen mit einem Kamm oder einer Bürste. Die Glättung kann auch unter Zuhilfenahme eines Glätteisens vorgenommen werden, wobei jedoch die hiermit verbundene Hitzeeinwirkung die Keratinsfasern zusätzlich schädigen können.

Eine weitere bevorzugte Ausführungsform ist daher ein Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und/oder das Färbemittel (F) mindestens ein Reduktionsmittel aus der Gruppe Thio-Milchsäure, Thio-Glycolsäure, Cystein, Natriumsulfid, Natriumsulfit, Natriumthiosulfat Natriumdithionit und/oder deren kosmetisch verträglichen Salze enthält,
- das Färbemittel (F) mindestens einen direktziehenden Säurefarbstoff enthält und
- die Glättung der Keratinfasern mechanisch in Schritt A) oder direkt nach Schritt A) erfolgt.

Wenn die Glättung in Schritt A) vorgenommen wird, bedeutet dies, dass die Glättung vorgenommen wird, während sich das Glättungsmittel (G) auf den Keratinfasern befindet. Erfolgt die Glättung direkt nach Schritt A), so wird die Glättung nach dem Abspülen des Glättungsmittels (G) und vor der Applikation des nächsten verfahrensgemäßen Mittels (d.h. vor der Applikation des Konditioniermittels (K) und/oder des Färbemittels (F)) vorgenommen.

Zwischen dem Auswaschen des Glättungsmittels (G) und der Applikation des Konditioniermittels (K) kann prinzipiell ein Zeitraum von bis zu 48 Stunden liegen. Es ist jedoch bevorzugt, wenn zwischen diesen beiden Schritten ein Zeitraum von maximal 24 Stunden, noch weiter bevorzugt von maximal 12 Stunden und besonders bevorzugt von maximal 30 Minuten liegt.

Zwischen dem Auswaschen des Konditioniermittels (K) und dem Auftragen des Färbemittels (F) kann ebenfalls prinzipiell ein Zeitraum von bis zu 48 Stunden liegen. Es ist jedoch bevorzugt, wenn zwischen diesen beiden Schritten ein Zeitraum von maximal 24 Stunden, noch weiter bevorzugt von maximal 12 Stunden und besonders bevorzugt von maximal 30 Minuten liegt.

Ganz besonders bevorzugt ist daher ein Verfahren des ersten Erfindungsgegenstands, umfassend die folgenden Schritte in der angegebenen Reihenfolge
A1) Applikation eines Glättungsmittels (G) auf die Fasern,
A2) Einwirkenlassen des Glättungsmittels (G) für einen Zeitraum von 30 Sekunden bis 45 Minuten,
A3) Ausspülen des Glättungsmittels (G),
B1) Applikation eines Konditioniermittels (K) auf die Fasern,
B2) Einwirkenlassen des Konditioniermittels (K) für einen Zeitraum von 30 Sekunden bis 45 Minuten,
B3) Ausspülen das Konditioniermittels (K),
C1) Applikation eines Färbemittels (F) aus die Fasern,
C2) Einwirkenlassen des Färbemittels für einen Zeitraum von 30 Sekunden bis 45 Minuten,
C3) Ausspülen das Färbemittels (F),
wobei
- zwischen den Schritten A3) und B1) ein zeitlicher Abstand von 10 Sekunden bis 24 Stunden, weiter bevorzugt von 10 Sekunden bis 12 Stunden und besonders bevorzugt von 10 Sekunden bis 30 Minuten liegt und
- zwischen den Schritten B3) und C1) ein zeitlicher Abstand von 10 Sekunden bis 24 Stunden, weiter bevorzugt von 10 Sekunden bis 12 Stunden und besonders bevorzugt von 10 Sekunden bis 30 Minuten liegt.

Weiterhin bevorzugt ist auch ein Verfahren, umfassend die folgenden Schritte in der angegebenen Reihenfolge
A1) Applikation eines Glättungsmittels (G) auf die Fasern,
A2) Einwirkenlassen des Glättungsmittels (G) für einen Zeitraum von 30 Sekunden bis 45 Minuten,
A3) Ausspülen des Glättungsmittels (G),
B1) Applikation eines Konditioniermittels (K) auf die Fasern,
B2) Einwirkenlassen des Konditioniermittels (K) für einen Zeitraum von 30 Sekunden bis 45 Minuten,
B3) Ausspülen das Konditioniermittels (K),
C1) Applikation eines Färbemittels (F) aus die Fasern,
C2) Einwirkenlassen des Färbemittels für einen Zeitraum von 30 Sekunden bis 45 Minuten,
C3) Ausspülen das Färbemittels (F),
wobei
- zwischen den Schritten A3) und B1) ein zeitlicher Abstand von 10 Sekunden bis 30 Minuten liegt und
- zwischen den Schritten B3) und C1) ein zeitlicher Abstand von 10 Sekunden bis 30 Minuten liegt.

Mit dem zeitlichen Mindestzeitraum von 10 Sekunden wurde der Zeitraum beziffert, den der Anwender üblicherweise mindestens benötigt, um nach Beendigung des Auswaschens des zuvor angewendeten Mittels das nächste Mittel zu greifen, dem Container, in dem es bereitgestellt wurde, zu entnehmen und auf die Keratinfasern zu applizieren.

Ein erfindungswesentliches Merkmal des Verfahrens zum Glätten und Färben der keratinischen Fasern besteht darin, dass das Konditioniermittel (K) und/oder das Färbemittel (F) mindestens ein Reduktionsmittel aus der Gruppe Thio-Milchsäure, Thio-Glycolsäure, Cystein, Natriumsulfid, Natriumsulfit, Natriumthiosulfat, Natriumdithionit und/oder deren kosmetisch verträglichen Salze enthält. Demnach kann entweder nur das Konditioniermittel (K) mindestens ein Reduktionsmittel aus der vorgenannten Gruppe enthalten, oder nur das Färbemittel (F) kann mindestens ein Reduktionsmittel aus der vorgenannten Gruppe enthalten, oder aber das Konditioniermittel (K) und das Färbemittel (F) enthalten beide mindestens ein Reduktionsmittel aus der vorgenannten Gruppe.

Bevorzugt ist daher auch ein Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) mindestens ein Reduktionsmittel aus der Gruppe Thio-Milchsäure, Thio-Glycolsäure, Cystein, Natriumsulfid, Natriumsulfit, Natriumthiosulfat, Natriumdithionit und/oder deren kosmetisch verträglichen Salze enthält und
- das Färbemittel (F) mindestens einen direktziehenden Säurefarbstoff enthält.

Weiterhin bevorzugt ist auch ein Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und das Färbemittel (F) unabhängig voneinander jeweils mindestens ein Reduktionsmittel aus der Gruppe Thio-Milchsäure, Thio-Glycolsäure, Cystein, Natriumsulfid, Natriumsulfit, Natriumthiosulfat, Natriumdithionit und/oder deren kosmetisch verträglichen Salze enthalten und
- das Färbemittel (F) mindestens einen direktziehenden Säurefarbstoff enthält.

Zur Anwendung des erfindungsgemäßen Verfahrens ist es für den Anwender besonders komfortabel und daher bevorzugt, wenn er sukzessive die Mittel anwendet, die für ihn in Form einer Mehrkomponenten-Verpackungseinheit (d.h. in Form eines Kits) bereitgestellt werden.

Ein zweiter Gegenstand der vorliegenden Erfindung ist daher eine Mehrkomponenten-Verpackungseinheit (Kit), umfassend drei getrennt voneinander konfektionierte kosmetische Mittel (G), (K) und (F), wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und/oder das Färbemittel (F) mindestens ein Reduktionsmittel aus der Gruppe Thio-Milchsäure, Thio-Glycolsäure, Cystein, Natriumsulfid, Natriumsulfit, Natriumthiosulfat, Natriumdithionit und/oder deren kosmetisch verträglichen Salzen enthält und
- das Färbemittel (F) mindestens einen direktziehenden Säurefarbstoff enthält.

Bevorzugt ist in diesem Zusammenhang eine Mehrkomponenten-Verpackungseinheit (Kit), umfassend drei getrennt voneinander konfektionierte kosmetische Mittel (G), (K) und (F), wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) mindestens ein Reduktionsmittel aus der Gruppe Thio-Milchsäure, Thio-Glycolsäure, Cystein, Natriumsulfid, Natriumsulfit, Natriumthiosulfat Natriumdithionit und/oder deren kosmetisch verträglichen Salzen enthält und
- das Färbemittel (F) mindestens einen direktziehenden Säurefarbstoff enthält.

Ebenfalls bevorzugt ist in diesem Zusammenhang eine Mehrkomponenten-Verpackungseinheit (Kit), umfassend drei getrennt voneinander konfektionierte kosmetische Mittel (G), (K) und (F), wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Färbemittel (F) mindestens ein Reduktionsmittel aus der Gruppe Thio-Milchsäure, Thio-Glycolsäure, Cystein, Natriumsulfid, Natriumsulfit, Natriumthiosulfat Natriumdithionit und/oder deren kosmetisch verträglichen Salzen enthält und
- das Färbemittel (F) mindestens einen direktziehenden Säurefarbstoff enthält.

Ebenfalls bevorzugt ist in diesem Zusammenhang eine Mehrkomponenten-Verpackungseinheit (Kit), umfassend drei getrennt voneinander konfektionierte kosmetische Mittel (G), (K) und (F), wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und das Färbemittel (F) unabhängig voneinander jeweils mindestens ein Reduktionsmittel aus der Gruppe Thio-Milchsäure, Thio-Glycolsäure, Cystein, Natriumsulfid, Natriumsulfit, Natriumthiosulfat Natriumdithionit und/oder deren kosmetisch verträglichen Salzen enthalten und
- das Färbemittel (F) mindestens einen direktziehenden Säurefarbstoff enthält.

Wenn das Konditioniermittel (K), dass im erfindungsgemäßen Verfahren verwendet oder in der erfindungsgemäßen Mehrkomponenten-Verpackungseinheit (Kit) bereitgestellt wird, mit einem oder mehreren Reduktionsmitteln versetzt wird, so hat dessen Anwendung eine überraschende Minimierung der Haarschädigung zur Folge. Die Minimierung der Haarschädigung manifestiert sich hierbei insbesondere in einer Reduktion des Cysteinsäuregehaltes des behandelten Haares.

Der Einsatz des bzw. der Reduktionsmittel in bestimmten Mengenbereichen hat sich in diesem Zusammenhang als besonders vorteilhaft im Hinblick auf die Verringerung der Haarschädigung erwiesen.

In einer besonders bevorzugten Ausführungsform ist ein erfindungsgemäßes Verfahren daher dadurch gekennzeichnet, dass das Konditioniermittel (K) ein oder mehrere Reduktionsmittel aus der Gruppe Thiomilchsäure, ThioGlycolsäure, Cystein und/oder deren physiologisch verträglichen Salzen in einer Gesamtmenge von 0,1 bis 10 Gew.-%, bevorzugt von 0,5 bis 8,5 Gew.-%, weiter bevorzugt von 1,5 bis 7,0 Gew.-% und besonders bevorzugt von 2,5 bis 6,5 Gew.-% - bezogen auf das Gesamtgewicht des Konditioniermittels (K) - enthält.

Weiterhin ist eine besonders bevorzugt Mehrkomponenten-Verpackungseinheit (Kit) dadurch gekennzeichnet, dass das Konditioniermittel (K) ein oder mehrere Reduktionsmittel aus der Gruppe Thiomilchsäure, ThioGlycolsäure, Cystein und/oder deren physiologisch verträglichen Salzen in einer Gesamtmenge von 0,1 bis 10 Gew.-%, bevorzugt von 0,5 bis 8,5 Gew.-%, weiter bevorzugt von 1,5 bis 7,0 Gew.-% und besonders bevorzugt von 2,5 bis 6,5 Gew.-% - bezogen auf das Gesamtgewicht des Konditioniermittels (K) - enthält.

Zusammenfassend ganz besonders bevorzugt ist demnach ein Verfahren des ersten Erfindungsgegenstandes und ein Kit des zweiten Erfindungsgegenstandes, dadurch gekennzeichnet, dass das Konditioniermittel (K) ein oder mehrere Reduktionsmittel aus der Gruppe Thiomilchsäure, ThioGlycolsäure, Cystein und/oder deren physiologisch verträglichen Salzen in einer Gesamtmenge von 0,1 bis 10 Gew.-%, bevorzugt von 0,5 bis 8,5 Gew.-%, weiter bevorzugt von 1,5 bis 7,0 Gew.-% und besonders bevorzugt von 2,5 bis 6,5 Gew.-% - bezogen auf das Gesamtgewicht des Konditioniermittels (K) - enthält.

Auch wenn das Färbemittel (F), dass im erfindungsgemäßen Verfahren verwendet oder in der erfindungsgemäßen Mehrkomponenten-Verpackungseinheit (Kit) bereitgestellt wird, mit einem oder mehreren Reduktionsmitteln versetzt wird, so hat dessen Anwendung eine überraschende Minimierung der Haarschädigung zur Folge. Die Minimierung der Haarschädigung manifestiert sich hierbei in analoger Weise in einer Reduktion des Cysteinsäuregehaltes des behandelten Haares. Der Einsatz des bzw. der Reduktionsmittel in bestimmten Mengenbereichen hat sich in diesem Zusammenhang als besonders vorteilhaft im Hinblick auf die Verringerung der Haarschädigung erwiesen.

In einer besonders bevorzugten Ausführungsform ist ein erfindungsgemäßes Verfahren daher dadurch gekennzeichnet, dass das Färbemittel (F) ein oder mehrere Reduktionsmittel aus der Gruppe Thiomilchsäure, ThioGlycolsäure, Cystein und/oder deren physiologisch verträglichen Salzen in einer Gesamtmenge von 1,2 bis 9 Gew.-%, bevorzugt von 1,6 bis 8,1 Gew.-%, weiter bevorzugt von 2,1 bis 6,6 Gew.-% und besonders bevorzugt von 2,6 bis 6,2 Gew.-% - bezogen auf das Gesamtgewicht des Färbemittels (F) - enthält.

Weiterhin ist eine besonders bevorzugte Mehrkomponenten-Verpackungseinheit (Kit) dadurch gekennzeichnet, dass das Färbemittel (F) ein oder mehrere Reduktionsmittel aus der Gruppe Thiomilchsäure, ThioGlycolsäure, Cystein und/oder deren physiologisch verträglichen Salzen in einer Gesamtmenge von 1,2 bis 9 Gew.-%, bevorzugt von 1,6 bis 8,1 Gew.-%, weiter bevorzugt von 2,1 bis 6,6 Gew.-% und besonders bevorzugt von 2,6 bis 6,2 Gew.-% - bezogen auf das Gesamtgewicht des Färbemittels (F) - enthält.

Zusammenfassend ganz besonders bevorzugt ist demnach ein Verfahren des ersten Erfindungsgegenstandes und ein Kit des zweiten Erfindungsgegenstandes, dadurch gekennzeichnet, dass das Färbemittel (F) ein oder mehrere Reduktionsmittel aus der Gruppe Thiomilchsäure, Thioglycolsäure, Cystein und/oder deren physiologisch verträgliche Salzen in einer Gesamtmenge von 1,2 bis 9 Gew.-%, bevorzugt von 1,6 bis 8,1 Gew.-%, weiter bevorzugt von 2,1 bis 6,6 Gew.-% und besonders bevorzugt von 2,6 bis 6,2 Gew.-% - bezogen auf das Gesamtgewicht des Färbemittels (F) - enthält. Unter Thiomilchsäure wird D-Thio-Milchsäure, L-Thio-Milchsäure und/oder deren Gemisch verstanden. Unter Cystein wird D-Cystein, L-Cystein und/oder deren Gemisch verstanden.

Ausgewählte Reduktionsmittel haben sich im Hinblick auf die Vermeidung bzw. Minimierung der Haarschädigung als ganz besonders effektiv erwiesen. Insbesondere bei Einsatz des Reduktionsmittels Ammoniumthiolactat konnten besonders geringe Cysteinsäure-Werte bei Vermessung der geglätteten und gefärbten Keratinfasern gemessen werden. Hierbei dient die Höhe des Gehaltes an Cysteinsäure in der Keratinfaser als Maß für den Ausprägungsgrad der Schädigung - je niedriger der Cysteinsäuregehalt ist, desto besser der Haarzustand.

Bei Ammoniumthiolactat handelt es sich um das Ammoniumsalz der Thiomilchsäure (d.h. das Ammoniumsalz der 2-Sulfanylpropionsäure).

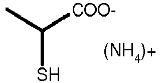

Von der Definition Ammoniumthiolactat mit umfasst sind sowohl die Ammoniumsalze der D-Thiomilchsäure als auch die Salze der L-Thiomilchsäure und deren Gemische.

In einer besonders bevorzugten Ausführungsform ist ein erfindungsgemäßes Verfahren daher dadurch gekennzeichnet, dass das Konditioniermittel (K) und/oder das Färbemittel (F) als Reduktionsmittel Ammoniumthiolactat in einer Menge von 2,7 bis 5,5 Gew.-% - jeweils bezogen auf das Gesamtgewicht des Konditioniermittels (K) und das Gesamtgewicht das Färbemittels (F) - enthält.

Weiterhin ist eine besonders bevorzugte Mehrkomponenten-Verpackungseinheit (Kit) dadurch gekennzeichnet, dass das Konditioniermittel (K) und/oder das Färbemittel (F) als Reduktionsmittel Ammoniumthiolactat in einer Menge von 2,7 bis 5,5 Gew.-% - jeweils bezogen auf das Gesamtgewicht des Konditioniermittels (K) und das Gesamtgewicht das Färbemittels (F) - enthält.

Zusammenfassend ganz besonders bevorzugt ist demnach ein Verfahren des ersten Erfindungsgegenstandes und ein Kit des zweiten Erfindungsgegenstandes, dadurch gekennzeichnet, dass das Konditioniermittel (K) und/oder das Färbemittel (F) als Reduktionsmittel Ammoniumthiolactat in einer Menge von 2,7 bis 5,5 Gew.-% enthält, wobei die Gewichtsangabe jeweils auf das Gesamtgewicht des Mittels bezogen ist, in dem das Ammoniumthiolactat eingesetzt wird.

Wenn das nur das Konditioniermittel (K) Ammoniumthiolactat enthält, erfolgt die Berechnung der im Konditioniermittel (K) enthaltenen Menge an Ammoniumthiolactat mit Bezug auf das Gesamtgewicht des Konditioniermittels (K). Wenn das nur das Färbemittel (F) Ammoniumthiolactat enthält, erfolgt die Berechnung der im Färbemittel (F) enthaltenen Menge an Ammoniumthiolactat mit Bezug auf das Gesamtgewicht des Färbemittels (F). Enthalten sowohl das Konditioniermittel (K) als auch das Färbemittel (F) Ammoniumthiolactat, so ist die Berechnungsgrundlage für die im Konditioniermittel (K) enthaltene Menge an Ammoniumthiolactat das Gesamtgewicht des Konditioniermittels (K) und für die im Färbemittel (F) enthaltene Menge an Ammoniumthiolactat das Gesamtgewicht des Färbemittels (F). Demzufolge ebenfalls bevorzugt ist ein erfindungsgemäßes Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- dass das Konditioniermittel (K) und/oder das Färbemittel (F) als Reduktionsmittel Ammoniumthiolactat in einer Menge von 2,7 bis 5,5 Gew.-% enthält, wobei die Gewichtsangabe jeweils auf das Gesamtgewicht des Mittels bezogen ist, in dem das Ammoniumthiolactat eingesetzt wird, und
- das Färbemittel (F) mindestens einen direktziehenden Säurefarbstoff enthält.

Weiterhin besonders bevorzugt ist ein erfindungsgemäßes Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) als Reduktionsmittel Ammoniumthiolactat in einer Menge von 2,7 bis 5,5 Gew.-% - jeweils bezogen auf das Gesamtgewicht des Konditioniermittels (K) - enthält.
- das Färbemittel (F) mindestens einen direktziehenden Säurefarbstoff enthält.

Weiterhin besonders bevorzugt ist ein erfindungsgemäßes Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Färbemittel (F) als Reduktionsmittel Ammoniumthiolactat in einer Menge von 2,7 bis 5,5 Gew.-%
- jeweils bezogen auf das Gesamtgewicht des Färbemittels (F) - enthält und
- das Färbemittel (F) mindestens einen direktziehenden Säurefarbstoff enthält.

Weiterhin besonders bevorzugt ist ein erfindungsgemäßes Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und das Färbemittel (F) unabhängig voneinander als Reduktionsmittel Ammoniumthiolactat in einer Menge von 2,7 bis 5,5 Gew.-% - jeweils bezogen auf das Gesamtgewicht des Konditioniermittels (K) und das Gesamtgewicht des Färbemittels (F) - enthalten und
- das Färbemittel (F) mindestens einen direktziehenden Säurefarbstoff enthält.

Weiterhin ist eine besonders bevorzugte Mehrkomponenten-Verpackungseinheit (Kit) dadurch gekennzeichnet, dass das Konditioniermittel (K) und/oder das Färbemittel (F) als Reduktionsmittel Ammoniumthiolactat in einer Menge von 2,7 bis 5,5 Gew.-% - jeweils bezogen auf das Gesamtgewicht des Konditioniermittels (K) und das Gesamtgewicht das Färbemittels (F) - enthält.

Weiterhin besonders bevorzugt ist eine erfindungsgemäße Mehrkomponenten-Verpackungseinheit (Kit), umfassend drei getrennt voneinander konfektionierte kosmetische Mittel (G), (K) und (F), wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) als Reduktionsmittel Ammoniumthiolactat in einer Menge von 2,7 bis 5,5 Gew.-% -jeweils bezogen auf das Gesamtgewicht des Konditioniermittels (K) enthält und
- das Färbemittel (F) mindestens einen direktziehenden Säurefarbstoff enthält.

Weiterhin besonders bevorzugt ist eine erfindungsgemäße Mehrkomponenten-Verpackungseinheit (Kit), umfassend drei getrennt voneinander konfektionierte kosmetische Mittel (G), (K) und (F), wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Färbemittel (F) als Reduktionsmittel Ammoniumthiolactat in einer Menge von 2,7 bis 5,5 Gew.-%
- jeweils bezogen auf das Gesamtgewicht des Färbemittels (F) - enthält und
- das Färbemittel (F) mindestens einen direktziehenden Säurefarbstoff enthält.

Weiterhin besonders bevorzugt ist eine erfindungsgemäße Mehrkomponenten-Verpackungseinheit (Kit), umfassend drei getrennt voneinander konfektionierte kosmetische Mittel (G), (K) und (F), wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und das Färbemittel (F) unabhängig voneinander als Reduktionsmittel Ammoniumthiolactat in einer Menge von 2,7 bis 5,5 Gew.-% - jeweils bezogen auf das Gesamtgewicht des Konditioniermittels (K) und das Gesamtgewicht das Färbemittels (F) - enthalten und
- das Färbemittel (F) mindestens einen direktziehenden Säurefarbstoff enthält.

Zusammenfassend ganz besonders bevorzugt ist ein Verfahren des ersten Erfindungsgegenstandes und ein Kit des zweiten Erfindungsgegenstandes, dadurch gekennzeichnet, dass das Konditioniermittel (K) und/oder das Färbemittel (F) als Reduktionsmittel Ammoniumthiolactat in einer Menge von 2,7 bis 5,5 Gew.-% enthält, wobei die Gewichtsangabe jeweils auf das Gesamtgewicht des Mittels bezogen ist, in dem das Ammoniumthiolactat eingesetzt wird.

Für das erfindungsgemäße Verfahren zum Glätten und Färben der Keratinfasern sowie für die entsprechende Mehrkomponenten-Verpackungseinheit ist weiterhin wesentlich, dass das Färbemittel (F) mindestens einen direktziehenden Säurefarbstoff enthält.

Direktziehende Farbstoffe können anhand ihrer Ladung in kationische Farbstoffe (basische Farbstoffe), nichtionische Farbstoffe und anionische Farbstoffe (auch Säurefarbstoffe genannt) unterteilt werden.

Unter Säurefarbstoffen werden direktziehende Farbstoffe verstanden, die mindestens eine Carbonsäuregruppierung (-COOH) und/oder eine Sulfonsäuregruppierung (-SO₃H) besitzen. In Abhängigkeit vom pH-Wert liegen die protonierten Formen (-COOH, -SO₃H) der Carbonsäure- bzw. Sulfonsäuregruppierungen mit ihren deprotonierten Formen (-COO⁻, -SO₃⁻ vor) im Gleichgewicht vor. Mit abnehmendem pH-Wert steigt der Anteil der protonierten Formen. Werden direktziehende Farbstoffe in Form ihrer Salze eingesetzt, so liegen die Carbonsäuregruppen bzw. Sulfonsäuregruppen in deprotonierter Form vor und sind zur Einhaltung der Elektroneutralität mit entsprechenden stöchiometrischen Äquivalente an Kationen (wie beispielsweise Na-Kation oder K-Kationen) neutralisiert.

Als geeignete Säurefarbstoffe können beispielsweise eine oder mehrere Verbindungen aus der folgenden Gruppe ausgewählt werden: Acid Yellow 1 (D&C Yellow 7, Citronin A, Ext. D&C Yellow No. 7, Japan Yellow 403, Cl 10316, COLIPA n° B001), Acid Yellow 3 (COLIPA n° : C 54, D&C Yellow Nº 10, Quinoline Yellow, E104, Food Yellow 13), Acid Yellow 9 (CI 13015), Acid Yellow 17 (CI 18965), Acid Yellow 23 (COLIPA n° C 29, Covacap Jaune W 1100 (LCW), Sicovit Tartrazine 85 E 102 (BASF), Tartrazine, Food Yellow 4, Japan Yellow 4, FD&C Yellow No. 5), Acid Yellow 36 (CI 13065), Acid Yellow 121 (CI 18690), Acid Orange 6 (CI 14270), Acid Orange 7 (2-Naphthol orange, Orange II, CI 15510, D&C Orange 4, COLIPA n° C015), Acid Orange 10 (C.I. 16230; Orange G sodium salt), Acid Orange 11 (CI 45370), Acid Orange 15 (CI 50120), Acid Orange 20 (CI 14600), Acid Orange 24 (BROWN 1;CI 20170;KATSU201;nosodiumsalt;Brown No.201;RESORCIN BROWN;ACID ORANGE 24;Japan Brown 201;D & C Brown No.1), Acid Red 14 (C.I.14720), Acid Red 18 (E124, Red 18; CI 16255), Acid Red 27 (E 123, CI 16185, C-Rot 46, Echtrot D, FD&C Red Nr.2, Food Red 9, Naphtholrot S), Acid Red 33 (Red 33, Fuchsia Red, D&C Red 33, CI 17200), Acid Red 35 (CI C.I.18065), Acid Red 51 (CI 45430, Pyrosin B, Tetraiodfluorescein, Eosin J, lodeosin), Acid Red 52 (CI 45100, Food Red 106, Solar Rhodamine B, Acid Rhodamine B, Red n° 106 Pontacyl Brilliant Pink), Acid Red 73 (CI CI 27290), Acid Red 87 (Eosin, CI 45380), Acid Red 95 (CI 45425, Erythtosine,Simacid Erythrosine Y), Acid Red 184 (CI 15685), Acid Red 195, Acid Violet 43 (Jarocol Violet 43, Ext. D&C Violet n° 2, C.I. 60730, COLIPA n° C063), Acid Violet 49 (CI 42640), Acid Violet 50 (CI 50325), Acid Blue 1 (Patent Blue, CI 42045), Acid Blue 3 (Patent Blau V, CI 42051), Acid Blue 7 (CI 42080), Acid Blue 104 (CI 42735), Acid Blue 9 (E 133, Patentblau AE, Amidoblau AE, Erioglaucin A, CI 42090, C.I. Food Blue 2), Acid Blue 62 (CI 62045), Acid Blue 74 (E 132, CI 73015), Acid Blue 80 (CI 61585), Acid Green 3 (CI 42085, Foodgreen1), Acid Green 5 (CI 42095), Acid Green 9 (C.I.42100), Acid Green 22 (C.I.42170), Acid Green 25 (CI 61570, Japan Green 201, D&C Green No. 5), Acid Green 50 (Brillantsäuregrün BS, C.I. 44090, Acid Brilliant Green BS, E 142), Acid Black 1 (Black n° 401, Naphthalene Black 10B, Amido Black 10B, CI 20 470, COLIPA n° B15), Acid Black 52 (CI 15711), Food Yellow 8 (CI 14270), Food Blue 5, D&C Yellow 8, D&C Green 5, D&C Orange 10,

D&C Orange 11, D&C Red 21, D&C Red 27, D&C Red 33, D&C Violet 2 und/oder D&C Brown 1.

Ein ganz besonders bevorzugtes Verfahren des ersten Erfindungsgegenstandes ist dadurch gekennzeichnet, dass das Färbemittel (F) mindestens einen direktziehenden Säurefarbstoff aus der Gruppe Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 und/oder D&C Brown 1 enthält.

Ein ganz besonders bevorzugter Kit des zweiten Erfindungsgegenstandes ist dadurch gekennzeichnet, dass das Färbemittel (F) mindestens einen direktziehenden Säurefarbstoff aus der Gruppe Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 und/oder D&C Brown 1 enthält.

Zusammenfassend ganz besonders bevorzugt ist ein Verfahren des ersten Erfindungsgegenstandes und ein Kit des zweiten Erfindungsgegenstandes, dadurch gekennzeichnet, dass das Färbemittel (F) mindestens einen direktziehenden Säurefarbstoff aus der Gruppe Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 und/oder D&C Brown 1 enthält.

Der bzw. die Säurefarbstoffe können bevorzugt in einer Gesamtmenge von 0,01 bis 5,5 Gew.-%, bevorzugt von 0,08 bis 4,7 Gew.-%, weiter bevorzugt von 0,2 bis 3,4 Gew.-% und besonders bevorzugt von 0,4 bis 1,8 Gew.-% im Färbemittel enthalten sein. Berechnungsgrundlage für die Gesamtmenge der Säurefarbstoffe ist hierbei das Gesamtgewicht des Färbmittels (F).

Weiterhin besonders bevorzugt ist daher ein Verfahren des ersten Erfindungsgegenstandes und ein Kit des zweiten Erfindungsgegenstandes, dadurch gekennzeichnet, dass das Färbemittel (F) den oder die direktziehenden Säurefarbstoffe in einer Gesamtmenge von 0,01 bis 5,5 Gew.-%, bevorzugt von 0,08 bis 4,7 Gew.-%, weiter bevorzugt von 0,2 bis 3,4 Gew.-% und besonders bevorzugt von 0,4 bis 1,8 Gew.-% - bezogen auf des Gesamtgewicht des Färbemittels (F) - enthält.

Das erfindungsgemäße Glättungsmittel (G) enthält eine oder mehrere Hydroxide aus der zuvor beschriebenen Gruppe. Hierbei handelt es sich um starke Basen. Dementsprechend besitzt auch das Glättungsmittel (G) einen alkalischen pH-Wert. Eine besonders gute Glättung wird dann erzielt, wenn das Glättungsmittel (G) einen pH-Wert von 8,5 bis 13,4, bevorzugt von 9,5 bis 13,3, weiter bevorzugt von 10,5 bis 13, 2 und besonders bevorzugt von 11,5 bis 13,0 besitzt.

Die Messung des pH-Wertes kann beispielsweise mit einer Glaselektrode erfolgen, die üblicherweise in Form einer Einstabmesskette ausgeführt ist. Bei den pH-Werten der vorliegenden Erfindung handelt es sich um pH-Werte, die bei einer Temperatur von 22 °C gemessen wurden.

Ein bevorzugtes Verfahren des ersten Erfindungsgegenstandes ist dadurch gekennzeichnet, dass das Glättungsmittel (G) einen pH-Wert von 8,5 bis 13,4, bevorzugt von 9,5 bis 13,3, weiter bevorzugt von 10,5 bis 13, 2 und besonders bevorzugt von 11,5 bis 13,0 besitzt.

Ein bevorzugter Kit des zweiten Erfindungsgegenstandes ist dadurch gekennzeichnet, dass das dass das Glättungsmittel (G) einen pH-Wert von 8,5 bis 13,4, bevorzugt von 9,5 bis 13,3, weiter bevorzugt von 10,5 bis 13, 2 und besonders bevorzugt von 11,5 bis 13,0 besitzt.

Zusammenfassend ganz besonders bevorzugt ist ein Verfahren des ersten Erfindungsgegenstandes und ein Kit des zweiten Erfindungsgegenstandes, dadurch gekennzeichnet, dass das Glättungsmittel (G) einen pH-Wert von 8,5 bis 13,4, bevorzugt von 9,5 bis 13,3, weiter bevorzugt von 10,5 bis 13, 2 und besonders bevorzugt von 11,5 bis 13,0 besitzt.

Auch das Konditioniermittel (K) wird vorteilhafterweise auf einen bestimmten pH-Wert eingestellt. Hierbei hat sich herausgestellt, dass der Einsatz von Konditioniermitteln (K), die einen pH-Wert zwischen 2,0 und 7,5 besitzen, im Hinblick auf die Reduzierung der Haarschädigung die meisten Vorteile aufweisen. Bevorzugt wird das Konditioniermittel auf einen pH-Wert von 3,0 bis 6,5 eingestellt, besonders geringe Schädigungen werden bei pH-Werten im Bereich von 3,5 bis 5,5 erhalten.

Ein bevorzugtes Verfahren des ersten Erfindungsgegenstandes ist dadurch gekennzeichnet, dass das Konditioniermittel (K) einen pH-Wert von 2,0 bis 7,5, bevorzugt von 2,5 bis 7,0, weiter bevorzugt von 3,0 bis 6,5 und besonders bevorzugt von 3,5 bis 5,5 besitzt.

Analog ist ein bevorzugter Kit des zweiten Erfindungsgegenstandes ist dadurch gekennzeichnet, dass das Konditioniermittel (K) einen pH-Wert von 2,0 bis 7,5, bevorzugt von 2,5 bis 7,0, weiter bevorzugt von 3,0 bis 6,5 und besonders bevorzugt von 3,5 bis 5,5 besitzt.

Zusammenfassend ganz besonders bevorzugt ist ein Verfahren des ersten Erfindungsgegenstandes und ein Kit des zweiten Erfindungsgegenstandes, dadurch gekennzeichnet, dass das Konditioniermittel (K) einen pH-Wert von 2,0 bis 7,5, bevorzugt von 2,5 bis 7,0, weiter bevorzugt von 3,0 bis 6,5 und besonders bevorzugt von 3,5 bis 5,5 besitzt.

Der pH-Wert des Färbemittels (F) liegt bevorzugt im sauren Bereich. Hierbei wirkt sich der saure pH-Bereich nicht nur im Hinblick auf die Farbintensität der mit den Säurefarbstoffen erhältlichen Färbung, sondern besonders auch im Hinblick auf die Reduzierung der Haarschädigung positiv aus. Bevorzugt liegt der pH-Wert des Färbemittels (F) bei einem Wert von 1,5 bis 6,0, bevorzugt von 1,6 bis 5,2, weiter bevorzugt von 1,7 bis 4,0 und besonders bevorzugt von 1,8 bis 2,5.

Ein bevorzugtes Verfahren des ersten Erfindungsgegenstandes ist dadurch gekennzeichnet, dass das Färbemittel (F) einen pH-Wert von 1,5 bis 6,0, bevorzugt von 1,6 bis 5,2, weiter bevorzugt von 1,7 bis 4,0 und besonders bevorzugt von 1,8 bis 2,5 besitzt.

Analog ist ein bevorzugter Kit des zweiten Erfindungsgegenstandes ist dadurch gekennzeichnet, dass das Färbemittel (F) einen pH-Wert von 1,5 bis 6,0, bevorzugt von 1,6 bis 5,2, weiter bevorzugt von 1,7 bis 4,0 und besonders bevorzugt von 1,8 bis 2,5 besitzt.

Zusammenfassend ganz besonders bevorzugt ist ein Verfahren des ersten Erfindungsgegenstandes und ein Kit des zweiten Erfindungsgegenstandes, dadurch gekennzeichnet, dass das Färbemittel (F) einen pH-Wert von 1,5 bis 6,0, bevorzugt von 1,6 bis 5,2, weiter bevorzugt von 1,7 bis 4,0 und besonders bevorzugt von 1,8 bis 2,5 besitzt.

Explizit ganz besonders bevorzugt ist ein erfindungsgemäßes Verfahren zum Glätten und Färben von keratinischen Fasern, umfassend die folgenden Schritte in der angegebenen Reihenfolge
A) Behandeln der Fasern mit einem Glättungsmittel (G),
B) Behandeln der Fasern mit einem Konditioniermittel (K),
C) Behandeln der Fasern mit einem Färbemittel (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält und einen pH-Wert von 11,5 bis 13,0 besitzt,
- das Konditioniermittel (K) und/oder das Färbemittel (F) als Reduktionsmittel Ammoniumthiolactat in einer Menge von 2,7 bis 5,5 Gew.-% - jeweils bezogen auf das Gesamtgewicht des Konditioniermittels (K) und das Gesamtgewicht das Färbemittels (F) - enthält,
- das Konditioniermittel (K) einen pH-Wert von 3,5 bis 5,5 besitzt,
- das Färbemittel (F) mindestens einen direktziehenden Säurefarbstoff enthält und einen pH-Wert von 1,8 bis 2,5 besitzt.

Bei dem Glättungsmittel (G) handelt es sich um ein Produkt auf Hydroxid-Basis, und das Glättungsmittel (G) ist selbst frei von Reduktionsmitteln.

Bevorzugt ist daher ein erfindungsgemäßes Verfahren und ein erfindungsgemäßer Kit, welche jeweils dadurch gekennzeichnet sind, dass das Glättungsmittel (G) frei von Reduktionsmitteln aus der Gruppe Thio-Milchsäure, Thio-Glycolsäure, Cystein, Natriumsulfid, Natriumsulfit, Natriumthiosulfat, Natriumdithionit und deren kosmetisch verträglichen Salzen ist.

Unter der Defintion "frei von Reduktionsmitteln" wird im Sinne der vorliegenden Erfindung verstanden, dass die Gesamtmenge der Reduktionsmittel aus der vorgenannten Gruppe weniger als 0,25 Gew.-%, bevorzugt weniger als 0,1 Gew.-% und besonders bevorzugt weniger als 0,05 Gew.-% - bezogen auf das Gesamtgewicht des Glättungsmittels (G) - beträgt.

Darüberhinaus ist es auch bevorzugt, wenn das Glättungsmittel auch von weiteren Reduktionsmitteln wie beispielsweise Ascorbinsäure frei ist. Damit ist es bevorzugt, wenn das Glättungsmittel (G) weniger als 0,25 Gew.-%, bevorzugt weniger als 0,1 Gew.-% und besonders bevorzugt weniger als 0,05 Gew.-% Ascorbinsäure - bezogen auf das Gesamtgewicht des Glättungsmittels (G) - enthält.

Ein bevorzugtes erfindungsgemäßes Verfahren sowie ein bevorzugter erfindungsgemäßer Kit sind daher weiterhin dadurch gekennzeichnet, dass das Glättungsmittel (G) frei von Reduktionsmitteln aus der Gruppe Thiomilchsäure, Thioglycolsäure, Cystein, Natriumsulfid, Natriumsulfit, Natriumthiosulfat, Natriumdithionit und deren Salzen ist.

Mit anderen Worten ist ein bevorzugtes erfindungsgemäßes Verfahren sowie ein bevorzugter erfindungsgemäßer Kit daher weiterhin dadurch gekennzeichnet, dass die Gesamtmenge der Reduktionsmittel aus der Gruppe Thio-Milchsäure, Thio-Glycolsäure, Cystein, Natriumsulfid, Natriumsulfit, Natriumthiosulfat, Natriumdithionit und deren Salzen im Glättungsmittel (G) weniger als 0,25 Gew.-%, bevorzugt weniger als 0,1 Gew.-% und besonders bevorzugt weniger als 0,05 Gew.-% - bezogen auf das Gesamtgewicht des Glättungsmittels (G) - beträgt.

Das Glättungsmittel (G), das Konditioniermittel (K) und/oder das Färbemittel (F) können weiterhin zusätzliche Wirk-, Hilfs- und Zusatzstoffe enthalten, um die Färbeleistung zu verbessern und weitere gewünschte Eigenschaften der Mittel einzustellen.

Vorzugsweise werden die Mittel (G), (K) und/oder (F) als flüssige Zubereitung bereitgestellt und den Mitteln daher gegebenenfalls zusätzlich eine weitere oberflächenaktive Substanz zugesetzt, wobei solche oberflächenaktive Substanzen je nach Anwendungsgebiet als Tenside oder als Emulgatoren bezeichnet werden: Sie sind bevorzugt aus anionischen, zwitterionischen, amphoteren und nichtionischen Tensiden und Emulgatoren ausgewählt.

Erfindungsgemäß geeignete Mittel sind dadurch gekennzeichnet, dass das Mittel zusätzlich mindestens ein anionisches Tensid enthält. Bevorzugte anionische Tenside sind Fettsäuren, Alkylsulfate, Alkylethersulfate und Ethercarbonsäuren mit 10 bis 20 C-Atomen in der Alkylgruppe und bis zu 16 Glykolethergruppen im Molekül. Die anionischen Tenside werden in Anteilen von 0,1 bis 45 Gew.%, bevorzugt 1 bis 30 Gew.% und ganz besonders bevorzugt von 1 bis 15 Gew.%, bezogen auf die Gesamtmenge des anwendungsbereiten Mittels, eingesetzt.

Erfindungsgemäß geeignete Mittel sind dadurch gekennzeichnet, dass das Mittel zusätzlich mindestens ein zwitterionisches Tensid enthält. Bevorzugte zwitterionische Tenside sind Betaine, N-Alkyl-N,N-dimethylammonium-glycinate, N-Acyl-aminopropyl-N,N-dimethylammoniumglycinate, und 2-Alkyl-3-carboxymethyl-3-hydroxyethyl-imidazoline. Ein bevorzugtes zwitterionisches Tensid ist unter der INCI-Bezeichnung Cocamidopropyl Betaine bekannt.

Erfindungsgemäß geeignete Mittel sind dadurch gekennzeichnet, dass das Mittel zusätzlich mindestens ein amphoteres Tensid enthält. Bevorzugte amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside sind N-Kokosalkylaminopropionat, as Kokosacylaminoethylaminopropionat und C₁₂-C₁₈-Acylsarcosin.

Weiterhin hat es sich als vorteilhaft erwiesen, wenn die Mittel weitere, nichtionogene grenzflächenaktive Stoffe, enthalten. Bevorzugte nichtionische Tenside sind Alkylpolyglykoside sowie Alkylenoxid-Anlagerungsprodukte an Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Die nichtionischen, zwitterionischen oder amphoteren Tenside werden in Anteilen von 0,1 bis 45 Gew.%, bevorzugt 1 bis 30 Gew.% und ganz besonders bevorzugt von 1 bis 15 Gew.%, jeweils bezogen auf die Gesamtmenge der Mittel (G), (K) und (F), eingesetzt.

Ebenfalls als vorteilhaft hat es sich erwiesen, wenn die Mittel mindestens ein Verdickungsmittel enthalten. Bezüglich dieser Verdickungsmittel bestehen keine prinzipiellen Einschränkungen. Es können sowohl organische als auch rein anorganische Verdickungsmittel zum Einsatz kommen. Geeignete Verdickungsmittel sind anionische, synthetische Polymere; kationische, synthetische Polymere; natürlich vorkommende Verdickungsmittel, wie nichtionische Guargums, Skleroglucangums oder Xanthangums, Gummi arabicum, Ghatti-Gummi, Karaya-Gummi, Tragant-Gummi, Carrageen-Gummi, Agar-Agar, Johannisbrotkernmehl, Pektine, Alginate, Stärke-Fraktionen und Derivate wie Amylose, Amylopektin und Dextrine, sowie Cellulosederivate, wie beispielsweise Methylcellulose, Carboxyalkylcellulosen und Hydroxyalkylcellulosen; nichtionische, vollsynthetische Polymere, wie Polyvinylalkohol oder Polyvinylpyrrolidinon; sowie anorganische Verdickungsmittel, insbesondere Schichtsilikate wie beispielsweise Bentonit, besonders Smektite, wie Montmorillonit oder Hectorit.

Die Mittel (G), (K) und/oder (F) können auch anionische polymere Verdickungsmittel enthalten. Geeignete Verbindungen sind beispielseweise ausgewählt aus den vernetzten oder unvernetzten Copolymeren, die mindestens zwei unterschiedliche Monomere aus der Gruppe der Acrylsäure, Methacrylsäure, den C₁-C₆-Alkylestern der Acrylsäure und/oder den C₁-C₆-Alkylestern der Methacrylsäure enthalten. Besonders bevorzugte anionische Copolymere sind Copolymere aus Acrylsäure, Methacrylsäure oder deren C₁-C₆-Alkylestern, die unter der INCI-Bezeichnung Acrylates Copolymer vertrieben werden. Insbesondere bevorzugt ist die Kombination aus Methacrylsäure und Ethylacrylat sowie gegebenenfalls vernetzenden, multifunktionalen Monomeren. Ein bevorzugtes Handelsprodukt dafür ist beispielsweise Aculyn® 33 bzw. 33A, das von der Firma Rohm & Haas angeboten wird.

Es ist weiterhin vorteilhaft, wenn das Konditioniermittel (K) mindestens ein kationisches Polymer und/oder ein kationisches Tensid enthält.

Ganz besonders bevorzugt ist daher auch ein Verfahren des ersten Erfindungsgegenstandes oder ein Kit des zweiten Erfindungsgegenstandes, dadurch gekennzeichnet, dass das Konditioniermittel (K) mindestens ein kationisches Polymer und/oder ein kationisches Tensid enthält.

Im Hinblick auf die Minimierung der Haarschädigung hat es sich insbesondere von Vorteil herausgestellt, wenn das Konditioniermittel (K) ein oder mehrere Polymere aus der Gruppe Polyquaternium-1, Polyquaternium-2, Polyquaternium-3, Polyquaternium-4, Polyquatenrium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquarternium-64, Polyquaternium-67, Polyquaternium-68, Polyquaternium-69 und/oder Polyquaternium-86 enthält.

Ganz besonders bevorzugt ist daher auch ein Verfahren des ersten Erfindungsgegenstandes oder ein Kit des zweiten Erfindungsgegenstandes, dadurch gekennzeichnet, dass das Konditioniermittel (K) ein oder mehrere Polymere aus der Gruppe Polyquaternium-1, Polyquaternium-2, Polyquaternium-3, Polyquaternium-4, Polyquatenrium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquarternium-64, Polyquaternium-67, Polyquaternium-68, Polyquaternium-69 und/oder Polyquaternium-86 enthält. Ganz besonders bevorzugt ist daher auch ein Verfahren des ersten Erfindungsgegenstandes und ein Kit des zweiten Erfindungsgegenstandes, dadurch gekennzeichnet, dass das Konditioniermittel (K) ein oder mehrere Polymere aus der Gruppe Polyquaternium-1, Polyquaternium-2, Polyquaternium-3, Polyquaternium-4, Polyquatenrium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquarternium-64, Polyquaternium-67, Polyquaternium-68, Polyquaternium-69 und/oder Polyquaternium-86 enthält. Erfindungsgemäß bevorzugte Verfahren und Kits sind dadurch gekennzeichnet, dass die Mittel (G), (K) und/oder (F) zusätzlich ein oder mehrere kationische Tenside enthalten. Als kationische Tenside können erfindungsgemäß alle dem Fachmann bekannten und übliche kationischen Tenside verwendet werden. Hierzu zählen:
- quartäre Imidazolinverbindung. Die im folgenden dargestellte Formel Quimi-I zeigt die Struktur dieser Verbindungen. 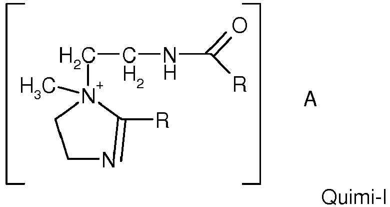 Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel I enthalten für R jeweils den gleichen Kohlenwasserstoffrest. Die Kettenlänge der Reste R beträgt bevorzugt 12 bis 21 Kohlenstoffatome. Besonders erfindungsgemäße Beispiele sind beispielsweise unter den INCII - Bezeichnungen Quaternium-27, Quaternium-72, Quaternium-83 und Quaternium-91 erhältlich.
- kationische Tenside gemäß der Formel (Tkat-2),

RCO-X-N+R¹R²R³ A- (Tkat-2)

R steht hierin für einen substituierten oder unsubstituierten, verzweigten oder geradkettigen Alkyl-oder Alkenylrest mit 11 bis 35 Kohlenstoffatomen in der Kette, X steht für - O - oder - NR⁵ -,
R¹ steht für eine Alkylengruppe mit 2 bis 6 C - Atomen, welche nicht substituiert oder substituiert sein kann, wobei im Falle einer Substitution die Substitution mit einer -OH - oder -NH- Gruppe bevorzugt ist,
R², R³ jeweils unabhängig voneinander stehen für eine Alkyl oder Hydroxyalkylgruppe mit 1 bis zu 6 C - Atomen in der Kette, wobei die Kette geradlinig oder verzweigt sein kann. R5 steht für Wasserstoff oder einen C1 bis C6 geradkettigen oder verzweigten, Alkyl- oder Alkenylrest, welcher auch durch eine Hydroxygruppe substituiert sein kann. Innerhalb dieser Strukturklasse werden bevorzugt die Verbindungen einer der folgenden Strukturen verwendet:

| | | |
|---|---|---|
| CH₃(CH₂)₂₀CONH(CH₂)₃ - N⁺(CH₃)₂-CH₂CH₃ | A- | (Tkat-3) |
| CH₃(CH₂)₂₀CONH(CH₂)₃-N+(CH₃)₂-CH₂(CHOH)CH₂OH | A- | (Tkat-4) |
| CH₃(CH2)₂₀COOCH₂CHOHCH₂ - N+(CH₃)₃ | A- | (Tkat-5) |
| CH₃(CH₂)₂₀CONH(CH₂)₃ - N+(CH₃)₂-CH₂CH₂OH | A- | (Tkat-6) |

Beispiele für derartige Handelsprodukte sind Schercoquat BAS, Lexquat AMG-BEO, Akypoquat 131 oder Incroquat Behenyl HE.
- Esterquats gemäß der Formel (Tkat1-2) verwendet werden. 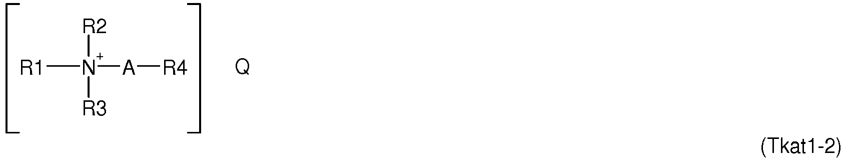 Hierin sind die Reste R1, R2 und R3 jeweils unabhängig voneinander und können gleich oder verschieden sein. Die Reste R1, R2 und R3 bedeuten:
- ein verzweigter oder unverzweigter Alkylrest mit 1 bis 4 Kohlenstoffatomen, welcher mindestens eine Hydroxylgruppe enthalten kann, oder
- ein gesättigter oder ungesättigter, verzweigter oder unverzweigter oder ein cyclischer gesättigter oder ungesättigter Alkylrest mit 6 bis 30 Kohlenstoffatomen, welcher mindestens eine Hydroxylgruppe enthalten kann, oder
- ein Aryl oder Alkarylrest, beispielsweise Phenyl oder Benzyl,
- den Rest (- A - R4), mit der Maßgabe, dass höchstens 2 der Reste R1, R2 oder R3 für diesen Rest stehen können: Der Rest -(A - R4) ist mindestens 1 bis 3 mal enthalten. Hierin steht A für:
   1) -(CH2)n- mit n = 1 bis 20, vorzugsweise n = 1 bis 10 und besonders bevorzugt n = 1 - 5, oder
   2) -(CH2-CHR5-O)n- mit n = 1 bis 200, vorzugsweise 1 bis 100, besonders bevorzugt 1 bis 50, und besonders bevorzugt 1 bis 20 mit R5 in der Bedeutung von Wasserstoff, Methyl oder Ethyl, und
   R4 steht für:
   1) R6-O-CO-, worin R6 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, oder
   2) R7-CO-, worin R7 einen gesättigten oder ungesättigten, verzweigten oder unverzweigten oder einen cyclischen gesättigten oder ungesättigten Alkylrest mit 6 bis 30 Kohlenstoffatomen ist, welcher mindestens eine Hydroxygruppe enthalten kann, und welcher gegebenenfalls weiterhin mit 1 bis 100 Ethylenoxideinheiten und/oder 1 bis 100 Propylenoxideinheiten oxethyliert sein kann, und
- Q steht für ein physiologisch verträgliches organisches oder anorganisches Anion.
   Solche Produkte werden beispielsweise unter den Warenzeichen Rewoquat®, Stepantex®, Dehyquart® und Armocare® vertrieben. Die Produkte Armocare® VGH-70, ein N,N-Bis(2-Palmitoyloxyethyl)dimethylammonium-chlorid, sowie Dehyquart® F-75, Dehyquart® C-4046, Dehyquart® L80, Dehyquart® F-30, Dehyquart® AU-35, Rewoquat® WE18, Rewoquat® WE38 DPG und Stepantex® VS 90 sind Beispiele für solche Esterquats.
Weitere erfindungsgemäß besonders bevorzugte Verbindungen der Formel (Tkat1-2) zählen zur Formel (Tkat1-2.1), den kationischen Betainestern. 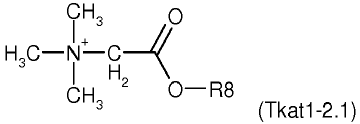 R8 entspricht in seiner Bedeutung R7.
- Monoalkyltrimethylammoniumsalze mit einer Kettenlänge des Alkylrestes von 16 bis 24 Kohlenstoffatomen entsprechend der Formel (Tkat1-1), 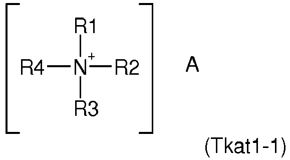 in denen R1, R2 und R3 für jeweils eine Methylgruppe stehen und R4 für einen gesättigten, verzweigten oder unverzweigten Alkylrest mit einer Kettenlänge von 16 bis 24 Kohlenstoffatomen. Beispiele für Verbindungen der Formel (Tkat1-1) sind Cetyltrimethylammoniumchlorid, Cetyltrimethylammoniumbromid, Cetyltrimethylammoniummethosulfat, Stearyltrimethylammoniumchlorid, Behenyltrimethylammoniumchlorid, Behenyltrimethylammoniumbromid und Behenyltrimethylammoniummethosulfat.
- Amine und/oder kationisiertes Amine, insbesondere ein Amidoamin und/oder ein kationisiertes Amidoamin mit den folgenden Strukturformeln:

R¹ - NH - (CH₂)ₙ - NR²R³ (Tkat7)

und/oder

R¹ - NH - (CH₂)ₙ - NR²R³R⁴ (Tkat8)

worin R1 ein Acyl-oder Alkylrest mit 6 bis 30 C-Atomen, welche verzweigt oder unverzweigt, gesättigt oder ungesättigt sein können, und wobei der Acylrest und/oder der Alkylrest mindestens eine OH-Gruppe enthalten können, und R2, R3 und R4 jeweils unabhängig voneinander Wasserstoff oder ein Alkylrest mit 1 bis 4 C-Atomen, welcher gleich oder verschieden, gesättigt oder ungesättigt sein kann, und X⁻ ein Anion und n eine ganze Zahl zwischen 1 und 10 bedeuten. Bevorzugt wird eine Zusammensetzung, in welcher das Amin und/oder das quaternisierte Amin gemäß allgemeiner Formeln (Tkat7) und/oder (Tkat8) ein Amidoamin und/oder ein quaternisiertes Amidoamin ist, worin R1 ein verzweigter oder unverzweigter, gesättigter oder ungesättigter Acylrest mit 6 bis 30 C-Atomen, welcher mindestens eine OH-Gruppe enthalten kann, bedeutet. Bevorzugt ist hierbei ein Fettsäurerest aus Ölen und Wachsen, insbesondere aus natürlichen Ölen und Wachsen, ist. Als Beispiele hierfür kommen Lanolin, Bienen-oder Candellilawachse in Betracht.Bevorzugt sind auch solche Amidoamine und/oder quaternisierte Amidoamine, in denen R2, R3 und/oder R4 in Formeln (Tkat7) und/oder (Tkat8) ein Rest gemäß der allgemeinen Formel CH₂CH₂OR5 bedeuten, worin R5 die Bedeutung von Alkylresten mit 1 bis 4 Kohlenstoffatomen, Hydroxyethyl oder Wasserstoff haben kann. Die bevorzugte Größe von n in den allgemeinen Formeln (Tkat7) und/oder (Tkat8) ist eine ganze Zahl zwischen 2 und 5. Weiterhin bevorzugt sind Amidoamine und/oder quaternisierte Amidoamine der allgemeinen Formeln (Tkat7) und/oder (Tkat8), in denen das Anion X- ein Halogenidion oder eine Verbindung der allgemeinen Formel RSO₃- ist, worin R die Bedeutung von gesättigtem oder ungesättigtem Alkylresten mit 1 bis 4 Kohlenstoffatomen hat. Der Alkylrest mit 1 bis 4 Kohlenstoffatomen von R2, R3 und R4 und/oder der Alkylrest mit 1 bis 4 Kohlenstoffatomen von RSO₃- in der allgemeinen Formel (Tkat7) und/oder (Tkat8) können mindestens eine Hydroxylgruppe enthalten. Die Alkylamidoamine können sowohl als solche vorliegen und durch Protonierung in entsprechend saurer Lösung in eine quaternäre Verbindung in der Zusammensetzung überführt werden. Erfindungsgemäß bevorzugt sind die kationischen Alkylamidoamine. Als erfindungsgemäß zu verwendende Amidoamine, welche gegebenenfalls quaternisiert sein können, kommen beispielsweise in Betracht als Amidoamine: Witcamine® 100 (Witco, INCI-Bezeichnung: Cocamidopropyl Dimethylamine), Incromine® BB (Croda, INCI-Bezeichnung: Behenamidopropyl Dimethylamine), Mackine® 401 (Mclntyre, INCI-Bezeichnung: Isostearylamidopropyl Dimethylamine) und andere Mackine-Typen, Adogen® S18V (Witco, INCI-Bezeichnung: Stearylamidopropyl Dimethylamine), und als permanent kationische Aminoamine: Rewoquat® RTM 50 (Witco Surfactants GmbH, INCI-Bezeichnung: Ricinoleamidopropyltrimonium Methosulfate), Empigen® CSC (Albright&Wilson, INCI-Bezeichnung: Cocamidopropyltrimonium Chlorid), Swanol® Lanoquat DES-50 (Nikko, INCI-Bezeichnung: Quatemium-33), Rewoquat® UTM 50 (Witco Surfactants GmbH, Undecyleneamidopropyltrimonium Methosulfate).

Das Anion der aller zuvor beschriebenen kationischen Verbindungen ist ausgewählt aus den physiologisch verträglichen Anionen. Beispielhaft hierfür seien die Halogenidionen, Fluorid, Chlorid, Bromid, Sulfat der allgemeinen Formel RSO₃-, worin R die Bedeutung von gesättigtem oder ungesättigtem Alkylresten mit 1 bis 4 Kohlenstoffatomen hat, oder anionische Reste organischer Säuren wie Maleat, Fumarat, Oxalat, Tartrat, Citrat, Lactat oder Acetat, genannt.

Bevorzugt werden kationische Imidazoline, Esterquats, kationische Tenside gemäß der Formel (Tkat-2) sowie Amine und/oder kationisiertes Amine, insbesondere Amidoamine und/oder kationisiertes Amidoamine verwendet.

Die zuvor genannten kationischen Tenside können einzeln oder in beliebigen Kombinationen miteinander verwendet werden, wobei Mengen zwischen 0,01 bis 20 Gew.%, bevorzugt in Mengen von 0,01 bis 10 Gew.% und ganz besonders bevorzugt in Mengen von 0,1 bis 7,5 Gew.% enthalten. Die allerbesten Ergebnisse werden dabei mit Mengen von 0,1 bis 5 Gew. % jeweils bezogen auf die Gesamtzusammensetzung des jeweiligen Mittels erhalten.

Die Tenside (T) werden in einer Gesamtmenge der Tenside in Mengen von 0,05 - 45 Gew.%, bevorzugt 0,1 - 30 Gew.% und ganz besonders bevorzugt von 0,5 - 25 Gew.%, bezogen auf das gesamte erfindungsgemäß verwendete Mittel, eingesetzt.

Die kationischen Tenside werden in Mengen von 0,1 bis 45 Gew.%, bevorzugt 1 bis 30 Gew.% und ganz besonders bevorzugt von 1 bis 15 Gew.% - jeweils bezogen auf die Gesamtmenge des jeweiligen Mittels (G), (K) und/oder (F) - eingesetzt.

Ferner können die erfindungsgemäßen Mittel weitere Wirk-, Hilfs- und Zusatzstoffe enthalten, wie beispielsweise Fettalkohole, nichtionische Polymere wie beispielsweise Vinylpyrrolidinon/Vinylacrylat-Copolymere, Polyvinylpyrrolidinon, Vinylpyrrolidinon/Vinylacetat-Copolymere, Polyethylenglykole und Polysiloxane; zusätzliche Silikone wie flüchtige oder nicht flüchtige, geradkettige, verzweigte oder cyclische, vernetzte oder nicht vernetzte Polyalkylsiloxane (wie Dimethicone oder Cyclomethicone), Polyarylsiloxane und/oder Polyalkylarylsiloxane, insbesondere Polysiloxane mit organofunktionelle Gruppen, wie substituierten oder unsubstituierten Aminen (Amodimethicone), Carboxyl-, Alkoxy- und/oder Hydroxylgruppen (Dimethiconcopolyole), lineare Polysiloxan(A)-Polyoxyalkylen(B)-Blockcopolymere, gepfropften Silikonpolymere; kationische Polymere wie quaternisierte Celluloseether, Polysiloxane mit quaternären Gruppen, Dimethyldiallylammoniumchlorid-Polymere, Acrylamid-Dimethyldiallyl-ammoniumchlorid-Copolymere, mit Diethylsulfat quaternierte Dimethylaminoethylmethacrylat-Vinylpyrrolidinon-Copolymere, Vinylpyrrolidinon-Imidazolinium-methochlorid-Copolymere und quaternierter Polyvinylalkohol; zwitterionische und amphotere Polymere; anionische Polymere wie beispielsweise Polyacrylsäuren oder vernetzte Polyacrylsäuren; Strukturanten wie Glucose, Maleinsäure und Milchsäure, haarkonditionierende Verbindungen wie Phospholipide, beispielsweise Lecitin und Kephaline; Parfümöle, Dimethylisosorbid und Cyclodextrine; faserstrukturverbessernde Wirkstoffe, insbesondere Mono-, Di- und Oligosaccharide wie beispielsweise Glucose, Galactose, Fructose, Fruchtzucker und Lactose; Farbstoffe zum Anfärben des Mittels; Antischuppenwirkstoffe wie Piroctone Olamine, Zink Omadine und Climbazol; Aminosäuren und Oligopeptide; Proteinhydrolysate auf tierischer und/oder pflanzlicher Basis, sowie in Form ihrer Fettsäure-Kondensationsprodukte oder gegebenenfalls anionisch oder kationisch modifizierten Derivate; pflanzliche Öle; Lichtschutzmittel und UV-Blocker; Wirkstoffe wie Panthenol, Pantothensäure, Pantolacton, Allantoin, Pyrrolidinoncarbonsäuren und deren Salze sowie Bisabolol; Polyphenole, insbesondere Hydroxyzimtsäuren, 6,7-Dihydroxycumarine, Hydroxybenzoesäuren, Catechine, Tannine, Leukoanthocyanidine, Anthocyanidine, Flavanone, Flavone und Flavonole; Ceramide oder Pseudoceramide; Vitamine, Provitamine und Vitaminvorstufen; Pflanzenextrakte; Fette und Wachse wie Fettalkohole, Bienenwachs, Montanwachs und Paraffine; Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate; Trübungsmittel wie Latex, Styrol/PVP- und Styrol/Acrylamid-Copolymere; Perlglanzmittel wie Ethylenglykolmono- und -distearat sowie PEG-3-distearat; Pigmente sowie Treibmittel wie Propan-Butan-Gemische, N₂O, Dimethylether, CO₂ und Luft. Die Auswahl dieser weiteren Stoffe wird der Fachmann gemäß der gewünschten Eigenschaften der Mittel treffen. Bezüglich weiterer fakultativer Komponenten sowie der eingesetzten Mengen dieser Komponenten wird ausdrücklich auf die dem Fachmann bekannten einschlägigen Handbücher verwiesen. Die zusätzlichen Wirk- und Hilfsstoffe werden in den erfindungsgemäßen Mitteln bevorzugt in Mengen von jeweils 0,0001 bis 25 Gew.-%, insbesondere von 0,0005 bis 15 Gew.-%, bezogen auf das Gesamtgewicht der Anwendungsmischung, eingesetzt.

Bei den zuvor beschriebenen erfindungsgemäßen Verfahren und Kits handelt es sich nicht um Verfahren, die einen Oxidationsschritt beinhalten. Das Verfahren grenzt sich daher von üblichen Dauerwell-Verfahren und oxidativen Färbeverfahren explizit ab, d.h. es kommt kein Wasserstoffperoxid zum Einsatz.

Zur Vermeidung von weiteren Haarschädigungen sind die erfindungsgemäßen Mittel (G), (K) und (F) daher auch frei von Oxidationsmitteln, insbesondere frei von Wasserstoffperoxid.

Ein bevorzugtes erfindungsgemäßes Verfahren sowie ein bevorzugter erfindungsgemäßer Kit sind daher weiterhin dadurch gekennzeichnet, dass
- die Gesamtmenge an Wasserstoffperoxid im Glättungsmittel (G) weniger als 0,1 Gew.-% - bezogen auf das Gesamtgewicht des Glättungsmittels (G) - beträgt
- die Gesamtmenge an Wasserstoffperoxid im Konditioniermittels (K) weniger als 0,1 Gew.-% - bezogen auf das Gesamtgewicht des Konditioniermittels (K) - beträgt und
- die Gesamtmenge an Wasserstoffperoxid im Färbemittel (F) weniger als 0,1 Gew.-% - bezogen auf das Gesamtgewicht des Färbemittels (F) - beträgt.

Es hat sich gezeigt, dass sich die Mehrkomponenten-Verpackungseinheiten des zweiten Erfindungsgegenstands sehr gut zum schädigungsarmen Glätten und Färben von Keratinsfasern eignen.

Ein weiterer Gegenstand der vorliegenden Erfindung ist daher die Verwendung einer Mehrkomponenten-Verpackungseinheit (Kits) des zweiten Erfindungsgegenstands in einem Verfahren des ersten Erfindungsgegenstands zur Verringerung der Schädigung beim Glätten und Färben von keratinischen Fasern.

Bezüglich weiterer bevorzugter Ausführungsformen des erfindungsgemäßen Verfahrens gilt mutatis mutandis das zu dem erfindungsgemäßen Verfahren und Kit Gesagte.

### Beispiele

Es wurden die folgenden Formulierungen hergestellt - alle Angaben erfolgen, sofern nicht anders angegeben, in Gewichtsprozent.

### 1. Glättungsmittel (G)

| Glättungsmittl (G) | Gew.-% |
|---|---|
| Paraffinum Liquidum | 10,0 |
| Cetearylalkohol (C₁₆/C₁₈ Fettalkohole) | 9,0 |
| Natriumcetearylsulfat | 1,0 |
| Proypylenglycol | 6,0 |
| Weichceresin FL 400 (Petrolatum) | 15,0 |
| Natriumhydroxid | 2,4 |
| Plantacare 2000 UP (Decylglucoside, 51 - 55 %ige Lsg. in Wasser | 0,8 |
| Laureth-4 | 1,0 |
| Polyquaternium-22 (39 - 43 %ige Lsg. in Wasser) | 0,10 |
| Plantasil Relaxcare ^{[1]} | 1,00 |
| Hydrolyzed Keratin | 0,30 |
| Bienenwachs | 0,50 |
| Aloe Vera Extrakt | 0,60 |
| Parfum | q.s. |
| Wasser (dest.) | ad 100 |

| | |
|---|---|
| ^{[1]} Plantasil Relaxcare, BASF, INCI: Potassium Silicates, Caprylyl/Capryl Glucoside, Glycerin | |

### 2. Konditioniermittel (K)

| Konditioniermittel (K) | K(V) Gew.-% (Vergleich) | K(E) Gew.-% erfindungsgemäß |
|---|---|---|
| Isopropylmyristat | 0,8 | 0,8 |
| Cetearylalkohol (C16/C18 Fettalkohole) | 3,0 | 3,0 |
| Dehyquart F75 ^{[2]} | 1,0 | 1,0 |
| Varisoft W 757 PG ^{[3]} | 4,0 | 4,0 |
| Citronensäure, Monohydrat | 0,5 | 0,5 |
| Stearamidopropyldimethylamin | 0,4 | 0,4 |
| Methylparaben | 0,3 | 0,3 |
| Cosmedia CTH ^{[4]} | 0,5 | 0,5 |
| Hydrolyzed Keratin | 0,1 | 0,1 |
| Croquat WKP PE LQ ^{[5]} | 0,1 | 0,1 |
| D-Panthenol (75 %ige Lsg. in Wasser) | 0,2 | 0,2 |
| Ammoniumthiolactat (70 %ige Lgs. in Wasser) | ---- | 5,0 |
| Phenoxyethanol | 0,4 | 0,4 |
| Parfum | q.s. | q.s. |
| Wasser | Ad 100 | Ad 100 |

| | | |
|---|---|---|
| ^{[2]} Dehyquart F75, BASF, INCI: DISTEAROYLETHYL HYDROXYETHYLMONIUM METHOSULFATE, CETEARYL ALCOHOL ^{[3]} Varisoft W 757 PG, Evonic, INCI: QUATERNIUM-27, PROPYLENE GLYCOL ^{[4]} Cosmedia CTH, BASF, INCI: POLYQUATERNIUM-37, PROPYLENE GLYCOL DICAPRY-LATE/DICAPRATE, PPG-1 TRIDECETH-6 ^{[5]} Croquat WKP PE LQ, Croda, INCI: Cocodimonium Hydroxypropyl Hydrolyzed Keratin (Solids 30.0-38.0%) | | |

### 3. Färbemittel (F)

| Färbemittel (F) | F(V) Gew.-% (Vergleich) | F(E) Gew.-% erfndungsgemäß |
|---|---|---|
| Ceteareth-12 | 2,6 | 2,6 |
| Phenoxyethanol | 0,81 | 0,81 |
| Etidronsäure (60 %ige Lsg. in Wasser) | 2,1 | 2,1 |
| Xanthan | 1,35 | 1,35 |
| Sepigel 305 [6] | 0,45 | 0,45 |
| Propylencarbonat | 5,0 | 5,0 |
| Acid Black No. 1 (CI 20470) | 0,2 | 0,2 |
| Acid Violet 43 (CI 60730, EXT. D&C VIOLET NO. 2) | 0,07 | 0,07 |
| Acid Orange 7 (D&C Orange No. 4, CI 15510) | 0,4 | 0,4 |
| Ammoniumthiolactat (70 %ige Lgs. in Wasser) | --- | 5,0 |
| Parfum | q.s. | q.s. |
| Wasser | Ad 100 | Ad 100 |

| | | |
|---|---|---|
| [6] Sepigel 305, Seppic, INCI: POLYACRYLAMIDE, C13-14 ISOPARAFFIN, LAURETH-7 (Solids 45-49%) | | |

### 4. Anwendung

Das Glättungsmittel (G) wurde auf Haarsträhnen (Kerling 6-0) gegeben, dort für 20 Minuten belassen und im Anschluss daran mit Wasser ausgespült. Im Anschluss daran wurde jeweils ein Konditioniermittel (K(V) bzw. K(E)) auf die Strähnen gegeben, dort für 10 Minuten belassen und im Anschluss daran mit Wasser ausgespült. Danach wurden die Strähnen jeweils für 40 Minuten mit einem Färbemittel (F(V) bzw. F(E)) behandelt. Nach der Behandlung wurde das Färbemittel wieder ausgespült.

### 5. Messung der Schädigung der behandelten Haarsträhnen

Zur Messung der durch die kombinierte Glättung und Färbung hervorgerufenen Haarschädigungen wurde der Cysteinsäurewert jeder behandelten Haarsträhne mittels quantitativer NIR-Spektroskopie bestimmt.
Die Aufnahme der Spektren erfolgte mit einem MPA™ ^{FT}-NIR Spetkrometer der Firma Bruker Optik GmbH. Der Infrarot-Bereich umfass den Wellenzahlbereich von 12500 cm⁻¹ bis 4000 cm⁻¹ und ist für Oberton- und Kombinationsschwingungen von z.B. CH-, OH- und NH-Gruppen charakteristisch.
Die Messung der Proben wurde mit dem Integrationskugelmodul an sechs verschiedenen Probenpositionen in diffuser Reflexion durchgeführt. Für die Analyse der gemessenen NIR-Spektren wurde der Wellenzahlbereich von 7300 cm⁻¹ bis 4020 cm⁻¹gewählt.
Die NIR Spektren von Cystin zeigen im Wellenzahlbereich von 6200 cm⁻¹ bis 5500 cm⁻¹ charakteristische Absorptionsbanden. Verändert sich das Haar durch stärkere Schädigung (d.h. nimmt der Cysteinsäuregehalt in den Haaren zu) wirkt sich dies im NIR-Spektrum auf die für Cysteinsäure charakteristischen Banden bei 5020 cm⁻¹ bis 4020 cm⁻¹ aus. Die quantitative Auswertung der NIR-Spektren erfolgte rechnergestützt.

Für jedes Glättungs-Färbe-Verfahren wurden jeweils drei Haarsträhnen behandelt und vermessen, aus diesen drei Messungen wurde jeweils der Mittelwert gebildet.

**NIR-Analysenwert [mol Cysteinsäure / 100 mol Aminosäure]**

| Nr. | Anwendung | | mol Cysteinsäure / 100 mol Aminosäure | Mittelwert |
|---|---|---|---|---|
| 1 | (G) + K(V) + F(V) | Vergleich, Konditioniermittel und Färbemittel ohne Ammoniumthiolactat | 1,2 | 1,3 |
| | | | 1,3 | |
| | | | 1,4 | |
| 2 | (G) + K(E) + F(V) | erfindungsgemäß, Konditioniermittel mit Ammoniumthiolactat | 1,2 | 1,1 |
| | | | 1,1 | |
| | | | 1,0 | |
| 3 | (G) + K(V) + F(E) | erfindungsgemäß, Färbemittel mit Ammoniumthiolactat | 1,1 | 1,1 |
| | | | 1,1 | |
| | | | 1,1 | |
| 4 | (G) + K(E) + F(E) | erfindungsgemäß, Konditioniermittel und Färbemittel mit Ammoniumthiolactat | 0,9 | 0,9 |
| | | | 0,9 | |
| | | | 0,9 | |

Die Haarsträhnen, die mit dem erfindungsgemäßen Verfahren bzw. mit dem erfindungsgemäßen Kit behandelt wurden (Versuche Nr. 2, 3 und 4) wiesen im Vergleich zum Verleichsversuch (Nr. 1) einen verminderten Cysteinsäuregehalt und damit eine verminderte Haarschädigung auf.

## Patentansprüche

1. Verfahren zum Glätten und Färben von keratinischen Fasern, umfassend die folgenden Schritte in der angegebenen Reihenfolge
A1) Applikation eines Glättungsmittels (G) auf die Fasern,
A2) Einwirkenlassen des Glättungsmittels (G) für einen Zeitraum von 30 Sekunden bis 45 Minuten.
A3) Ausspülen des Glättungsmittels (G),
B1) Applikation eines Konditioniermittels (K) auf die Fasern,
B2) Einwirkenlassen des Konditioniermittels (K) für einen Zeitraum von 30 Sekunden bis 45 Minuten,
B3) Ausspülen das Konditioniermittels (K),
C1) Applikation eines Färbemittels (F) aus die Fasern,
C2) Einwirkenlassen des Färbemittels für einen Zeitraum von 30 Sekunden bis 45 Minuten.
C3) Ausspülen das Färbemittels (F),
wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und/oder das Färbemittel (F) mindestens ein Reduktionsmittel aus der Gruppe Thio-Milchsäure, Thio-Glycolsäure, Cystein, Natriumsulfid, Natriumsulfit, Natriumthiosulfat, Natriumdithionit und/oder deren kosmetisch verträglichen Salze enthält und
- das Färbemittel (F) mindestens einen direktziehenden Säurefarbstoff enthält, und
- zwischen den Schritten A3) und B1) ein zeitlicher Abstand von 10 Sekunden bis 48 Stunden liegt und
- zwischen den Schritten B3) und C1) ein zeitlicher Abstand von 10 Sekunden bis 48 Stunden liegt.

2. Verfahren nach Anspruch 1, wobei
- zwischen den Schritten A3) und B1) ein zeitlicher Abstand von 10 Sekunden bis 24 Stunden, weiter bevorzugt von 10 Sekunden bis 12 Stunden und besonders bevorzugt von 10 Sekunden bis 30 Minuten liegt und
- zwischen den Schritten B3) und C1) ein zeitlicher Abstand von 10 Sekunden bis 24 Stunden, weiter bevorzugt von 10 Sekunden bis 12 Stunden und besonders bevorzugt von 10 Sekunden bis 30 Minuten liegt.

3. Mehrkomponenten-Verpackungseinheit (Kit), umfassend drei getrennt voneinander konfektionierte kosmetische Mittel (G), (K) und (F), wobei
- das Glättungsmittel (G) mindestens ein Alkalisierungsmittel aus der Gruppe Natriumhydroxid, Kaliumhydroxid, Lithiumhydroxid, Magnesiumhydroxid, Calciumhydroxid und/oder Guanidiniumhydroxid enthält,
- das Konditioniermittel (K) und/oder das Färbemittel (F) mindestens ein Reduktionsmittel aus der Gruppe Thio-Milchsäure, Thio-Glycolsäure, Cystein, Natriumsulfid, Natriumsulfit, Natriumthiosulfat, Natriumdithionit und/oder deren kosmetisch verträglichen Salzen enthält und
- das Färbemittel (F) mindestens einen direktziehenden Säurefarbstoff enthält.

4. Verfahren nach einem der Ansprüche 1 bis 2 oder Kit nach Anspruch 3, **dadurch gekennzeichnet, dass** das Konditioniermittel (K) ein oder mehrere Reduktionsmittel aus der Gruppe Thiomilchsäure, Thioglycolsäure, Cystein und/oder deren physiologisch verträglichen Salze in einer Gesamtmenge von 0,1 bis 10 Gew.-%, bevorzugt von 0,5 bis 8,5 Gew.-%, weiter bevorzugt von 1,5 bis 7,0 Gew.-% und besonders bevorzugt von 2,5 bis 6,5 Gew.-% - bezogen auf das Gesamtgewicht des Konditioniermittels (K) - enthält.

5. Verfahren oder Kit nach einem der Ansprüche 1 bis 4, **dadurch gekennzeichnet, dass** das Färbemittel (F) ein oder mehrere Reduktionsmittel aus der Gruppe Thiomilchsäure, ThioGlycolsäure, Cystein und/oder deren physiologisch verträglichen Salze in einer Gesamtmenge von 1,2 bis 9 Gew.-%, bevorzugt von 1,6 bis 8,1 Gew.-%, weiter bevorzugt von 2,1 bis 6,6 Gew.-% und besonders bevorzugt von 2,6 bis 6,2 Gew.-% - bezogen auf das Gesamtgewicht des Färbemittels (F) - enthält.

6. Verfahren oder Kit nach einem der Ansprüche 1 bis 5, **dadurch gekennzeichnet, dass** das Konditioniermittel (K) und/oder das Färbemittel (F) als Reduktionsmittel Ammoniumthiolactat in einer Menge von 2,7 bis 5,5 Gew.-% enthält, wobei die Gewichtsangabe jeweils auf das Gesamtgewicht des Mittels bezogen ist, in dem das Ammoniumthiolactat eingesetzt wird.

7. Verfahren oder Kit nach einem der Ansprüche 1 bis 6, **dadurch gekennzeichnet, dass** das Färbemittel (F) mindestens einen direktziehenden Säurefarbstoff aus der Gruppe Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 und/oder D&C Brown 1 enthält.

8. Verfahren oder Kit nach einem der Ansprüche 1 bis 7, **dadurch gekennzeichnet, dass** das Färbemittel (F) den oder die direktziehenden Säurefarbstoffe in einer Gesamtmenge von 0,01 bis 5,5 Gew.-%, bevorzugt von 0,08 bis 4,7 Gew.-%, weiter bevorzugt von 0,2 bis 3,4 Gew.-% und besonders bevorzugt von 0,4 bis 1,8 Gew.-% - bezogen auf des Gesamtgewicht des Färbemittels (F) - enthält.

9. Verfahren oder Kit nach einem der Ansprüche 1 bis 8, **dadurch gekennzeichnet, dass** das Glättungsmittel (G) einen pH-Wert von 8,5 bis 13,4, bevorzugt von 9,5 bis 13,3, weiter bevorzugt von 10,5 bis 13, 2 und besonders bevorzugt von 11,5 bis 13,0 besitzt.

10. Verfahren oder Kit nach einem der Ansprüche 1 bis 9, **dadurch gekennzeichnet, dass** das Konditioniermittel (K) einen pH-Wert von 2,0 bis 7,5, bevorzugt von 2,5 bis 7,0, weiter bevorzugt von 3,0 bis 6,5 und besonders bevorzugt von 3,5 bis 5,5 besitzt.

11. Verfahren oder Kit nach einem der Ansprüche 1 bis 10, **dadurch gekennzeichnet, dass** das Färbemittel (F) einen pH-Wert von 1,5 bis 6,0, bevorzugt von 1,6 bis 5,2, weiter bevorzugt von 1,7 bis 4,0 und besonders bevorzugt von 1,8 bis 2,5 besitzt.

12. Verfahren oder Kit nach einem der Ansprüche 1 bis 11, **dadurch gekennzeichnet, dass** das Glättungsmittel (G) frei von Reduktionsmitteln aus der Gruppe Thio-Milchsäure, Thio-Glycolsäure, Cystein, Natriumsulfid, Natriumsulfit, Natriumthiosulfat, Natriumdithionit und deren Salzen ist.

13. Verfahren oder Kit nach einem der Ansprüche 1 bis 12, **dadurch gekennzeichnet, dass** das Konditioniermittel (K) ein oder mehrere Polymere aus der Gruppe Polyquaternium-1, Polyquaternium-2, Polyquaternium-3, Polyquaternium-4, Polyquatenrium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquarternium-64, Polyquaternium-67, Polyquaternium-68, Polyquaternium-69 und/oder Polyquaternium-86 enthält.

14. Verwendung einer Mehrkomponenten-Verpackungseinheit nach einem der Ansprüche 3 bis 13 in einem Verfahren nach einem der Ansprüche 1 bis 13 zur Verringerung der Schädigung beim Glätten und Färben von keratinischen Fasern.

## Claims

1. A method for straightening and dyeing keratin fibers, comprising the following steps in the stated sequence
A1) applying a straightening agent (G) to the fibers,
A2) allowing the straightening agent (G) to act for a period of 30 seconds to 45 minutes,
A3) rinsing out the straightening agent (G),
B1) applying a conditioner (K) to the fibers,
B2) allowing the conditioner (K) to act for a period of 30 seconds to 45 minutes,
B3) rinsing out the conditioner (K),
C1) applying a coloring agent (F) to the fibers,
C2) allowing the coloring agent to act for a period of 30 seconds to 45 minutes,
C3) rinsing out the coloring agent (F),
wherein
- the straightening agent (G) contains at least one alkalizing agent from the group consisting of sodium hydroxide, potassium hydroxide, lithium hydroxide, magnesium hydroxide, calcium hydroxide and/or guanidinium hydroxide,
- the conditioner (K) and/or the coloring agent (F) contains at least one reducing agent from the group consisting of thiolactic acid, thioglycolic acid, cysteine, sodium sulfide, sodium sulfite, sodium thiosulfate, sodium dithionite and/or cosmetically acceptable salts thereof and
- the coloring agent (F) contains at least one direct acid dye, and
- a time interval of 10 seconds to 48 hours is left between steps A3) and B1) and
- a time interval of 10 seconds to 48 hours is left between steps B3) and C1).

2. The method according to claim 1,
wherein
- a time interval of 10 seconds to 24 hours, more preferably 10 seconds to 12 hours, and particularly preferably 10 seconds to 30 minutes, is left between steps A3) and B1), and
- a time interval of 10 seconds to 24 hours, more preferably 10 seconds to 12 hours, and particularly preferably 10 seconds to 30 minutes, is left between steps B3) and C1).

3. A multicomponent packaging unit (kit), comprising three separately packaged cosmetic agents (G), (K) and (F), wherein
- the straightening agent (G) contains at least one alkalizing agent from the group consisting of sodium hydroxide, potassium hydroxide, lithium hydroxide, magnesium hydroxide, calcium hydroxide and/or guanidinium hydroxide,
- the conditioner (K) and/or the coloring agent (F) contains at least one reducing agent from the group consisting of thiolactic acid, thioglycolic acid, cysteine, sodium sulfide, sodium sulfite, sodium thiosulfate, sodium dithionite and/or cosmetically acceptable salts thereof and
- the coloring agent (F) contains at least one direct acid dye.

4. The method according to one of claims 1 to 2 or the kit according to claim 3, **characterized in that** the conditioner (K) contains one or more reducing agents from the group consisting of thiolactic acid, thioglycolic acid, cysteine and/or physiologically acceptable salts thereof in a total amount of from 0.1 to 10 wt.%, preferably from 0.5 to 8.5 wt.%, more preferably from 1.5 to 7.0 wt.%, and particularly preferably from 2.5 to 6.5 wt.%, based on the total weight of the conditioner (K).

5. The method or kit according to one of claims 1 to 4, **characterized in that** the coloring agent (F) contains one or more reducing agents from the group consisting of thiolactic acid, thioglycolic acid, cysteine and/or physiologically acceptable salts thereof in a total amount of from 1.2 to 9 wt.%, preferably from 1.6 to 8.1 wt.%, more preferably from 2.1 to 6.6 wt.%, and particularly preferably from 2.6 to 6.2 wt.%, based on the total weight of the coloring agent (F).

6. The method or kit according to one of claims 1 to 5, **characterized in that** the conditioner (K) and/or the coloring agent (F) contains ammonium thiolactate as the reducing agent in an amount of from 2.7 to 5.5 wt.%, the weight being given in each case based on the total weight of the agent in which the ammonium thiolactate is used.

7. The method or kit according to one of claims 1 to 6, **characterized in that** the coloring agent (F) contains at least one direct acid dye from the group consisting of Acid Yellow 1, Acid Yellow 3, Acid Yellow 9, Acid Yellow 17, Acid Yellow 23, Acid Yellow 36, Acid Yellow 121, Acid Orange 6, Acid Orange 7, Acid Orange 10, Acid Orange 11, Acid Orange 15, Acid Orange 20, Acid Orange 24, Acid Red 14, Acid Red 18, Acid Red 27, Acid Red 33, Acid Red 35, Acid Red 51, Acid Red 52, Acid Red 73, Acid Red 87, Acid Red 95, Acid Red 184, Acid Red 195, Acid Violet 43, Acid Violet 49, Acid Violet 50, Acid Blue 1, Acid Blue 3, Acid Blue 7, Acid Blue 104, Acid Blue 9, Acid Blue 62, Acid Blue 74, Acid Blue 80, Acid Green 3, Acid Green 5, Acid Green 9, Acid Green 22, Acid Green 25, Acid Green 50, Acid Black 1, Acid Black 52, Food Yellow 8, Food Blue 5, D&C Yellow 7, D&C Yellow 8, D&C Orange 4, D&C Green 5, D&C Orange 10, D&C Orange 11, D&C Red 21, D&C Red 27, D&C Red 33, D&C Violet 2 and/or D&C Brown 1.

8. The method or kit according to one of claims 1 to 7, **characterized in that** the coloring agent (F) contains the direct acid dye(s) in a total amount of from 0.01 to 5.5 wt.%, preferably from 0.08 to 4.7 wt.%, more preferably from 0.2 to 3.4 wt.% and particularly preferably from 0.4 to 1.8 wt.%, based on the total weight of the coloring agent (F).

9. The method or kit according to one of claims 1 to 8, **characterized in that** the straightening agent (G) has a pH of from 8.5 to 13.4, preferably from 9.5 to 13.3, more preferably from 10.5 to 13.2, and particularly preferably from 11.5 to 13.0.

10. The method or kit according to one of claims 1 to 9, **characterized in that** the conditioner (K) has a pH of from 2.0 to 7.5, preferably from 2.5 to 7.0, more preferably from 3.0 to 6.5, and particularly preferably from 3.5 to 5.5.

11. The method or kit according to one of claims 1 to 10, **characterized in that** the coloring agent (F) has a pH of from 1.5 to 6.0, preferably from 1.6 to 5.2, more preferably from 1.7 to 4.0, and particularly preferably from 1.8 to 2.5.

12. The method or kit according to one of claims 1 to 11, **characterized in that** the straightening agent (G) is free of reducing agents from the group consisting of thiolactic acid, thioglycolic acid, cysteine, sodium sulfide, sodium sulfite, sodium thiosulfate, sodium dithionite and salts thereof.

13. The method or kit according to one of claims 1 to 12, **characterized in that** the conditioner (K) contains one or more polymers from the group consisting of Polyquaternium-1, Polyquaternium- 2, Polyquaternium-3, Polyquaternium-4, Polyquaternium-5, Polyquaternium-6, Polyquaternium-7, Polyquaternium-8, Polyquaternium-9, Polyquaternium-10, Polyquaternium-11, Polyquaternium-14, Polyquaternium-16, Polyquaternium-17, Polyquaternium-18, Polyquaternium-22, Polyquaternium-24, Polyquaternium-27, Polyquaternium-28, Polyquaternium-32, Polyquaternium-33, Polyquaternium-37, Polyquaternium-39, Polyquaternium-44, Polyquaternium-46, Polyquaternium-53, Polyquaternium-55, Polyquarternium-64, Polyquaternium-67, Polyquaternium-68, Polyquaternium-69 and/or Polyquaternium-86.

14. The use of a multicomponent packaging unit according to one of claims 3 to 13 in a method according to one of claims 1 to 13 for reducing damage when straightening and dyeing keratin fibers.

## Revendications

1. Procédé pour lisser et colorer les fibres kératiniques, comprenant les étapes suivantes dans l'ordre indiqué
A1) Application d'un agent de lissage (G) sur les fibres.
A2) Laisser agir l'agent de lissage (G) pendant une période de 30 secondes à 45 minutes,
A3) Rinçage de l'agent de lissage (G),
B1) Application d'un agent de conditionnement (K) sur les fibres,
B2) Laisser agir l'agent de conditionnement (K) pendant une période de 30 secondes à 45 minutes,
B3) Rinçage de l'agent de conditionnement (K),
C1) Application d'un agent de coloration (F) sur les fibres,
C2) Laisser agir l'agent de coloration pendant une période de 30 secondes à 45 minutes,
C3) Rinçage de l'agent de coloration (F),
dans lequel
- l'agent de lissage (G) contient au moins un agent d'alcalinisation choisi dans le groupe comprenant l'hydroxyde de sodium, l'hydroxyde de potassium, l'hydroxyde de lithium, l'hydroxyde de magnésium, l'hydroxyde de calcium et/ou l'hydroxyde de guanidinium,
- l'agent de conditionnement (K) et/ou l'agent de coloration (F) contient au moins un agent de réduction choisi dans le groupe comprenant l'acide thiolactique, l'acide thioglycolique, la cystéine, le sulfure de sodium, le sulfite de sodium, le thiosulfate de sodium, le dithionite de sodium et/ou leurs sels cosmétiquement acceptables et
- l'agent de coloration (F) contient au moins un colorant acide direct, et
- un intervalle de temps de 10 secondes à 48 heures s'écoule entre les étapes A3) et B1) et
- un intervalle de temps de 10 secondes à 48 heures s'écoule entre les étapes B3) et C1).

2. Procédé selon la revendication 1, dans lequel
- un intervalle de temps de 10 secondes à 24 heures s'écoule entre les étapes A3) et B1), plus préférablement de 10 secondes à 12 heures, et de manière particulièrement préférée de 10 secondes à 30 minutes, et
- un intervalle de temps de 10 secondes à 24 heures s'écoule entre les étapes B3) et C1), plus préférablement de 10 secondes à 12 heures, et de manière particulièrement préférée de 10 secondes à 30 minutes.

3. Unité d'emballage à plusieurs composants (kit), comprenant trois produits cosmétiques (G), (K) et (F) confectionnés séparément, dans lequel
- l'agent de lissage (G) contient au moins un agent d'alcalinisation choisi dans le groupe comprenant l'hydroxyde de sodium, l'hydroxyde de potassium, l'hydroxyde de lithium, l'hydroxyde de magnésium, l'hydroxyde de calcium et/ou l'hydroxyde de guanidinium,
- l'agent de conditionnement (K) et/ou l'agent de coloration (F) contient au moins un agent de réduction choisi dans le groupe comprenant l'acide thiolactique, l'acide thioglycolique, la cystéine, le sulfure de sodium, le sulfite de sodium, le thiosulfate de sodium, le dithionite de sodium et/ou leurs sels cosmétiquement acceptables et
- l'agent de coloration (F) contient au moins un colorant acide direct.

4. Procédé selon l'une des revendications 1 à 2 ou kit selon la revendication 3, **caractérisé en ce que** l'agent de conditionnement (K) contient un ou plusieurs agent(s) de réduction choisi(s) dans le groupe comprenant l'acide thiolactique, l'acide thioglycolique, la cystéine et/ou leurs sels physiologiquement acceptables à raison d'une quantité totale de 0,1 à 10% en poids, de préférence de 0,5 à 8,5% en poids, plus préférablement de 1,5 à 7,0% en poids et de manière particulièrement préférée de 2,5 à 6,5% en poids - par rapport au poids total de l'agent de conditionnement (K).

5. Procédé ou kit selon l'une des revendications 1 à 4, **caractérisé en ce que** l'agent de coloration (F) contient un ou plusieurs agent(s) de réduction choisi(s) dans le groupe comprenant l'acide thiolactique, l'acide thioglycolique, la cystéine et/ou leurs sels physiologiquement acceptables à raison d'une quantité totale de 1,2 à 9% en poids, de préférence de 1,6 à 8,1% en poids, plus préférablement de 2,1 à 6,6% en poids et de manière particulièrement préférée de 2,6 à 6,2% en poids - par rapport au poids total de l'agent de coloration (F).

6. Procédé ou kit selon l'une des revendications 1 à 5, **caractérisé en ce que** l'agent de conditionnement (K) et/ou l'agent de coloration (F) contien(nen)t en tant qu'agent(s) de réduction du thiolactate d'ammonium à raison d'une quantité de 2,7 à 5,5% en poids, dans lequel la donnée de poids est dans chaque cas basée sur le poids total de l'agent dans lequel le thiolactate d'ammonium est utilisé.

7. Procédé ou kit selon l'une des revendications 1 à 6, **caractérisé en ce que** l'agent de coloration (F) contient au moins un colorant acide direct choisi dans le groupe comprenant l'Acid Yellow 1, l'Acid Yellow 3, l'Acid Yellow 9, l'Acid Yellow 17, l'Acid Yellow 23, l'Acid Yellow 36, l'Acid Yellow 121, l'Acid Orange 6, l'Acid Orange 7, l'Acid Orange 10, l'Acid Orange 11, l'Acid Orange 15, l'Acid Orange 20, l'Acid Orange 24, l'Acid Red 14, l'Acid Red 18, l'Acid Red 27, l'Acid Red 33, l'Acid Red 35, l'Acid Red 51, l'Acid Red 52, l'Acid Red 73, l'Acid Red 87, l'Acid Red 95, l'Acid Red 184, l'Acid Red 195, l'Acid Violet 43, l'Acid Violet 49, l'Acid Violet 50, l'Acid Blue 1, l'Acid Blue 3, l'Acid Blue 7, l'Acid Blue 104, l'Acid Blue 9, l'Acid Blue 62, l'Acid Blue 74, l'Acid Blue 80, l'Acid Green 3, l'Acid Green 5, l'Acid Green 9, l'Acid Green 22, l'Acid Green 25, l'Acid Green 50, l'Acid Black 1, l'Acid Black 52, le Food Yellow 8, le Food Blue 5, le D&C Yellow 7, le D&C Yellow 8, le D&C Orange 4, le D&C Green 5, le D&C Orange 10, le D&C Orange 11, le D&C Red 21, le D&C Red 27, le D&C Red 33, le D&C Violet 2 et/ou le D&C Brown 1

8. Procédé ou kit selon l'une des revendications 1 à 7, **caractérisé en ce que** l'agent de coloration (F) contient le(s) colorant(s) acide(s) direct(s) à raison d'une quantité totale de 0,01 à 5,5% en poids, de préférence de 0,08 à 4,7% en poids, plus préférablement de 0,2 à 3,4% en poids et de manière particulièrement préférée de 0,4 à 1,8% en poids - par rapport au poids total de l'agent de coloration (F).

9. Procédé ou kit selon l'une des revendications 1 à 8, **caractérisé en ce que** l'agent de lissage (G) a un pH de 8,5 à 13,4, de préférence de 9,5 à 13,3, plus préférablement de 10,5 à 13,2 et de manière particulièrement préférée de 11,5 à 13,0.

10. Procédé ou kit selon l'une des revendications 1 à 9, **caractérisé en ce que** l'agent de conditionnement (K) a un pH de 2,0 à 7,5, de préférence de 2,5 à 7,0, plus préférablement de 3,0 à 6,5 et de manière particulièrement préférée de 3,5 à 5,5.

11. Procédé ou kit selon l'une des revendications 1 à 10, **caractérisé en ce que** l'agent de coloration (F) a un pH de 1,5 à 6,0, de préférence de 1,6 à 5,2, plus préférablement de 1,7 à 4,0 et de manière particulièrement préférée de 1,8 à 2,5.

12. Procédé ou kit selon l'une des revendications 1 à 11, **caractérisé en ce que** l'agent de lissage (G) est exempt d'agents de réduction du groupe comprenant l'acide thiolactique, l'acide thioglycolique, la cystéine, le sulfure de sodium, le sulfite de sodium, le thiosulfate de sodium, le dithionite de sodium et leurs sels.

13. Procédé ou kit selon l'une des revendications 1 à 12, **caractérisé en ce que** l'agent de conditionnement (K) contient un ou plusieurs polymères choisi dans le groupe comprenant le Polyquaternium-1, le Polyquaternium-2, le Polyquaternium-3, le Polyquaternium-4, le Polyquatenrium-5, le Polyquaternium- 6, le Polyquaternium-7, le Polyquaternium-8, le Polyquaternium-9, le Polyquaternium-10, le Polyquaternium-11, le Polyquaternium-14, le Polyquaternium-16, le Polyquaternium-17, le Polyquaternium-18, le Polyquaternium-22, le Polyquaternium-24, le Polyquaternium-27, le Polyquaternium-28, le Polyquaternium-32, le Polyquaternium-33, le Polyquaternium-37, le Polyquaternium-39, le Polyquaternium-44, le Polyquaternium-46, le Polyquaternium-53, le Polyquaternium-55, le Polyquaternium-64, le Polyquaternium-67, le Polyquaternium-68, le Polyquaternium-69 et/ou le Polyquaternium-86.

14. Utilisation d'une unité d'emballage à plusieurs composants selon l'une des revendications 3 à 13 dans un procédé selon l'une des revendications 1 à 13 pour réduire les dommages lors du lissage et de la coloration des fibres kératiniques.
